# EUROPEAN PATENT SPECIFICATION

(11) **EP 1 390 477 B1**
(45) Date of publication and mention of the grant of the patent: **16.08.2006**
(21) Application number: 01991129.6
(22) Date of filing: 30.10.2001
(51) Int. Cl.: C12N 9/00

(54) **POLYNUCLEOTIDES ENCODING HUMAN MELTRIN BETA (ADAM19) METALLOENDOPEPTIDASES**
POLYNUKLEOTIDE DIE FÜR HUMANE MELTRIN BETA (ADAM19) METALLOENDOPEPTIDASEN KODIEREN
NOUVELLES PROTEASES HUMAINES ET POLYNUCLEOTIDES CODANT POUR LESDITES PROTEASES

(30) Priority: 01.11.2000 US 244939 P
(43) Date of publication of application: 25.02.2004
(73) Proprietor: Lexicon Genetics Incorporated, The Woodlands, TX 77381-1160 (US)
(72) Inventor: WALKE, D., Wade, Spring, TX 77389 (US); WILGANOWSKI, Nathaniel, L., Texas, TX 77388 (US); FRIDDLE, Carl, Johan, The Woodlands, TX 77382 (US)
(74) Representative: Silveston, Judith
(86) International application number: PCT/US2001/048498
(87) International publication number: WO 2002/036759

(56) References cited:
- WO-A-97/09420
- FRITSCHE, J. ET AL.: "Molecular cloning and characterization of a human metalloprotease disintegrin - a novel marker for dendritic cell differentiation" BLOOD, vol. 96, no. 2, - 15 July 2000 (2000-07-15) XP001119962
- DATABASE EMBL [Online] ADAM gene 1, 20 March 2001 (2001-03-20) RUBEN ET AL.: retrieved from EMBL Database accession no. AAC91177 XP002236360 & WO 00 73323 A (HUMAN GENOME SCIENCES) 7 December 2000 (2000-12-07)
- WEI P ET AL: "EXPRESSION AND ENZYMATIC ACTIVITY OF HUMAN DISINTEGRIN AND METALLOPROTEINASE ADAM19/MELTRIN BETA" BIOCHEMICAL AND BIOPHYSICAL RESEARCH COMMUNICATIONS, ACADEMIC PRESS INC. ORLANDO, FL, US, vol. 280, no. 3, 26 January 2001 (2001-01-26), pages 744-755, XP001120634 ISSN: 0006-291X

## Description

### 1. INTRODUCTION

The present invention relates to the discovery, identification, and characterization of novel human polynucleotides encoding proteins sharing sequence similarity with mammalian meltrin-beta\ADAM 19 homologue metalloproteases , The invention encompasses the described polynucleotides, host cell expression systems, the encoded proteins, fusion proteins, polypeptides and peptides, antibodies to the encoded proteins and peptides, and genetically engineered animals that either lack or over express the disclosed sequences, antagonists and agonists of the proteins, and other compounds that modulate the expression or activity of the proteins encoded by the disclosed polynucleotides that can be used for diagnosis, drug screening, clinical trial monitoring, the treatment of diseases and disorders, and cosmetic or nutriceutical applications.

### 2. BACKGROUND OF THE INVENTION

Proteases cleave protein substrates as part of degradation, maturation, and secretory pathways within the body. Proteases have been associated with, inter alia, regulating development, diabetes, obesity, infertility, modulating cellular processes, and infectious disease.

### 3. SUMMARY OF THE INVENTION

The present invention relates to the discovery, identification, and characterization of nucleotides that encode novel human proteins, and the corresponding amino acid sequences of these proteins. The novel human proteins (NHPs) described for the first time herein share structural similarity with animal proteases and particularly metalloproteases and disintegrins such as meltrin-beta and ADAM 19.

The novel human nucleic acid (cDNA) sequences described herein, encode proteins/open reading frames (ORFs) of 926, 918, 963, and 955 amino acids in length (see SEQ ID NOS: 2, 4, 6, and 8 respectively).

The invention also encompasses agonists and antagonists of the described NHPs, including small molecules, large molecules, mutant NHPs, or portions thereof, that compete with native NHP, peptides, and antibodies, as well as nucleotide sequences that can be used to inhibit the expression of the described NHPs (e.g., antisense and ribozyme molecules, and open reading frame or regulatory sequence replacement constructs) or to enhance the expression of the described NHPs (e.g., expression constructs that place the described polynucleotide under the control of a strong promoter system), and non-human transgenic animals that express a NHP sequence, or "knock-outs" (which can be conditional) that do not express a functional NHP. Knock-out mice can be produced in several ways, one of which involves the use of mouse embryonic stem cells ("ES cells") lines that contain gene trap mutations in a murine homolog of at least one of the described NHPs. When the unique NHP sequences described in SEQ ID NOS:1-9 are "knocked-out" they provide a method of identifying phenotypic expression of the particular gene as well as a method of assigning function to previously unknown genes. In addition, animals in which the unique NHP sequences described in SEQ ID NOS:1-9 are "knocked-out" provide a unique source in which to elicit antibodies to homologous and orthologous proteins which would have been previously viewed by the immune system as "self" and therefore would have failed to elicit significant antibody responses. To these ends, gene trapped knockout ES cells have been generated in murine homologs of the described NHPs.

Additionally, the unique NHP sequences described in SEQ ID NOS:1-9 are useful for the identification of protein coding sequence and mapping a unique gene to a particular chromosome (the gene encoding the described NHPs is apparently encoded on human chromosome 5, see GENBANK accession no. AC008676). These sequences identify actual, biologically relevant, exon splice junctions as opposed to those that might have been predicted bioinformatically from genomic sequence alone. The sequences of the present invention are also useful as additional DNA markers for restriction fragment length polymorphism (RFLP) analysis, and in forensic biology.

Further, the present invention also relates to processes for identifying compounds that modulate, i.e., act as agonists or antagonists, of NHP expression and/or NHP activity that utilize purified preparations of the described NHPs and/or NHP product, or cells expressing the same. Such compounds can be used as therapeutic agents for the treatment of any of a wide variety of symptoms associated with biological disorders or imbalances.

### 4. DESCRIPTION OF THE SEQUENCE LISTING AND FIGURES

The Sequence Listing provides the sequences of several NHP ORFs encoding the described NHP amino acid sequences. SEQ ID NO:9 describes a NHP ORF and flanking sequences.

### 5. DETAILED DESCRIPTION OF THE INVENTION

The NHP sequences described for the first time herein are novel proteins that are expressed in, inter alia, human cell lines, and human fetal brain, brain, pituitary, cerebellum, spinal cord, thymus, spleen, lymph node, bone marrow, trachea, lung, kidney, fetal liver, liver, prostate, testis, adrenal gland, pancreas, salivary gland, stomach, small intestine, colon, skeletal muscle, heart, uterus, placenta, mammary gland, skin, adipose, esophagus, bladder, cervix, rectum, hypothalamus, ovary, fetal kidney, gall bladder, tongue, carcinoma cells, umbilical vein, endothelium, and fetal lung cells.

The described sequences were compiled from cDNA clones, genomic sequence, and cDNAs derived from human fetal brain, testis, mammary gland, placenta, adipose, uterus, skeletal muscle, fetus, kidney, brain, thymus, and adrenal gland mRNAs (Edge Biosystems, Gaithersburg, MD, and Clontech, Palo Alto, CA). The present invention encompasses the nucleotides presented in the Sequence Listing, host cells expressing such nucleotides, the expression products of such nucleotides, and: (a) nucleotides that encode mammalian homologs of the described genes, including the specifically described NHPs, and NHP products; (b) nucleotides that encode one or more portions of a NHP that correspond to functional domains, and the polypeptide products specified by such nucleotide sequences, including but not limited to the novel regions of any active domain(s); (c) isolated nucleotides that encode mutant versions, engineered or naturally occurring, of the described NHPs in which all or a part of at least one domain is deleted or altered, and the polypeptide products specified by such nucleotide sequences, including but not limited to soluble proteins and peptides in which all or a portion of the signal sequence is deleted; (d) nucleotides that encode chimeric fusion proteins containing all or a portion of a coding region of a NHP, or one of its domains (e.g., a receptor or ligand binding domain, accessory protein/self-association domain, etc.) fused to another peptide or polypeptide; or (e) therapeutic or diagnostic derivatives of the described polynucleotides such as oligonucleotides, anti sense- po-lynuc-l-eetides, ribozymes, dsRNA, or gene-therapy constructs comprising a sequence first disclosed in the Sequence Listing.

As discussed above, the present invention includes: (a) the human DNA sequences presented in the Sequence Listing (and vectors comprising the same) and additionally contemplates any nucleotide sequence encoding a contiguous NHP open reading frame (ORF), or a contiguous exon splice junction first described in the Sequence Listing, that hybridizes to a complement of a DNA sequence presented in the Sequence Listing under highly stringent conditions, e.g., hybridization to filter-bound DNA in 0.5 M NaHPO₄, 7% sodium dodecyl sulfate (SDS), 1 mM EDTA at 65°C, and washing in 0.1xSSC/0.1% SDS at 68°C (Ausubel F.M. *et al.,* eds., 1989, Current Protocols in Molecular Biology, Vol. I, Green Publishing Associates, Inc., and John Wiley & Sons, Inc., New York, at p. 2.10.3) and encodes a functionally equivalent expression product. Additionally contemplated are any nucleotide sequences that hybridize to the complement of the DNA sequence that encode and express an amino acid sequence presented in the Sequence Listing under moderately stringent conditions, e.g., washing in 0.2xSSC/0.1% SDS at 42°C (Ausubel et *al.,* 1989, *supra),* yet still encode a functionally equivalent NHP product. Functional equivalents of a NHP include naturally occurring NHPs present in other species and mutant NHPs whether naturally occurring or engineered (by site directed mutagenesis, gene shuffling, directed evolution as described in, for example, U.S. Patent No. 5,837,458). The invention also includes degenerate nucleic acid variants of the disclosed NHP polynucleotide sequences.

Additionally contemplated are polynucleotides encoding a NHP ORF, or its functional equivalent, encoded by a polynucleotide sequence that is about 99, 95, 90, or about 85 percent similar or identical to corresponding regions of the nucleotide sequences of the Sequence Listing (as measured by BLAST sequence comparison analysis using, for example, the GCG sequence analysis package using standard default settings).

The invention also includes nucleic acid molecules, preferably DNA molecules, that hybridize to, and are therefore the complements of, the described NHP gene nucleotide sequences. Such hybridization conditions may be highly stringent or less highly stringent, as described above. In instances where the nucleic acid molecules are deoxyoligonucleotides ("DNA oligos"), such molecules are generally about 16 to about 100 bases long, or about 20 to about 80, or about 34 to about 45 bases long, or any variation or combination of sizes represented therein that incorporate a contiguous region of sequence first disclosed in the Sequence Listing. Such oligonucleotides can be used in conjunction with the polymerase chain reaction (PCR) to screen libraries, isolate clones, and prepare cloning and sequencing templates, etc.

Alternatively, such NHP oligonucleotides can be used as hybridization probes for screening libraries, and assessing gene expression patterns (particularly using a micro array or high-throughput "chip" format). Additionally, a series of the described NHP oligonucleotide sequences, or the complements thereof, can be used to represent all or a portion of the described NHP sequences. An oligonucleotide or polynucleotide sequence first disclosed in at least a portion of one or more of the sequences of SEQ ID NOS: 1-9 can be used as a hybridization probe in conjunction with a solid support matrix/substrate (resins, beads, membranes, plastics, polymers, metal or metallized substrates, crystalline or polycrystalline substrates, etc.). Of particular note are spatially addressable arrays (i.e., gene chips, microtiter plates, etc.) of oligonucleotides and polynucleotides, or corresponding oligopeptides and polypeptides, wherein at least one of the biopolymers present on the spatially addressable array comprises an oligonucleotide or polynucleotide sequence first disclosed in at least one of the sequences of SEQ ID NOS: 1-9, or an amino acid sequence encoded thereby. Methods for attaching biopolymers to, or synthesizing biopolymers on, solid support matrices, and conducting binding studies thereon are disclosed in, *inter alia,* U.S. Patent Nos. 5,700,637, 5,556,752, 5,744,305, 4,631,211, 5,445,934, 5,252,743, 4,713,326, 5,424,186, and 4,669,405.

Addressable arrays comprising sequences first disclosed in SEQ ID NOS:1-9 can be used to identify and characterize the temporal and tissue specific expression of a gene. These addressable arrays incorporate oligonucleotide sequences of sufficient length to confer the required specificity, yet be within the limitations of the production technology. The length of these probes is within a range of between about 8 to about 2000 nucleotides. Preferably the probes consist of 60 nucleotides, and more preferably 25 nucleotides, from the sequences first disclosed in SEQ ID NOS: 1-9.

For example, a series of the described oligonucleotide sequences, or the complements thereof, can be used in chip format to represent all or a portion of the described sequences. The oligonucleotides, typically between about 16 to about 40 (or any whole number within the stated range) nucleotides in length can partially overlap each other and/or the sequence may be represented using oligonucleotides that do not overlap. Accordingly, the described polynucleotide sequences shall typically comprise at least about two or three distinct oligonucleotide sequences of at least about 8 nucleotides in length that are each first disclosed in the described Sequence Listing. Such oligonucleotide sequences can begin at any nucleotide present within a sequence in the Sequence Listing and proceed in either a sense (5'-to-3') orientation vis-a-vis the described sequence or in an antisense orientation.

Microarray-based analysis allows the discovery of broad patterns of genetic activity, providing new understanding of gene functions and generating novel and unexpected insight into transcriptional processes and biological mechanisms. The use of addressable arrays comprising sequences first disclosed in SEQ ID NOS:1-9 provides detailed information about transcriptional changes involved in a specific pathway, potentially leading to the identification of novel components or gene functions that manifest themselves as novel phenotypes.

Probes consisting of sequences first disclosed in SEQ ID NOS:1-9 can also be used in the identification, selection and validation of novel molecular targets for drug discovery. The use of these unique sequences permits the direct confirmation of drug targets and recognition of drug dependent changes in gene expression that are modulated through pathways distinct from the drugs intended target. These unique sequences therefore also have utility in defining and monitoring both drug action and toxicity.

As an example of utility, the sequences first disclosed in SEQ ID NOS:1-9 can be utilized in microarrays or other assay formats, to screen collections of genetic material from patients who have a particular medical condition. These investigations can also be carried out using the sequences first disclosed in SEQ ID NOS:1-9 *in silico* and by comparing previously collected genetic databases and the disclosed sequences using computer software known to those in the art.

Thus,the sequences first disclosed in SEQ ID NOS:1-9 can be used to identify mutations associated with a particular disease and also as a diagnostic or prognostic assay.

Although the presently described sequences have been specifically described using nucleotide sequence, it should be appreciated that each of the sequences can uniquely be described using any of a wide variety of additional structural attributes, or combinations thereof. For example, a given sequence can be described by the net composition of the nucleotides present within a given region of the sequence in conjunction with the presence of one or more specific oligonucleotide sequence(s) first disclosed in the SEQ ID NOS: 1-9. Alternatively, a restriction map specifying the relative positions of restriction endonuclease digestion sites, or various palindromic or other specific oligonucleotide sequences can be used to structurally describe a given sequence. Such restriction maps, which are typically generated by widely available computer programs (e.g., the University of Wisconsin GCG sequence analysis package, SEQUENCHER 3.0, Gene Codes Corp., Ann Arbor, MI, etc.), can optionally be used in conjunction with one or more discrete nucleotide sequence(s) present in the sequence that can be described by the relative position of the sequence relative to one or more additional sequence(s) or one or more restriction sites present in the disclosed sequence.

For oligonucleotide probes, highly stringent conditions may refer, e.g., to washing in 6xSSC/0.05% sodium pyrophosphate at 37°C (for 14-base oligos), 48°C (for 17-base oligos), 55°C (for 20-base oligos), and 60°C (for 23-base oligos). These nucleic acid molecules may encode or act as NHP gene antisense molecules, useful, for example, in NHP gene regulation (for and/or as antisense primers in amplification reactions of NHP gene nucleic acid sequences). With respect to NHP gene regulation, such techniques can be used to regulate biological functions. Further, such sequences may be used as part of ribozyme and/or triple helix sequences that are also useful for NHP gene regulation.

Inhibitory antisense or double stranded oligonucleotides can additionally comprise at least one modified base moiety which is selected from the group, including but not limited to, 5-fluorouracil, 5-bromouracil, 5-chlorouracil, 5-iodouracil, hypoxanthine, xantine, 4-acetylcytosine, 5-(carboxyhydroxylmethyl) uracil, 5-carboxymethylaminomethyl-2-thiouridine, 5-carboxymethylaminomethyluracil, dihydrouracil, beta-D-galactosylqueosine, inosine, N6-isopentenyladenine, 1-methylguanine, 1-methylinosine, 2,2-dimethylguanine, 2-methyladenine, 2-methylguanine, 3-methylcytosine, 5-methylcytosine, N6-adenine, 7-methylguanine, 5-methylaminomethyluracil, 5-methoxyaminomethyl-2-thiouracil, beta-D-mannosylqueosine, 5'-methoxycarboxymethyluracil, 5-methoxyuracil, 2-methylthio-N6-isopentenyladenine, uracil-5-oxyacetic acid (v), wybutoxosine, pseudouracil, queosine, 2-thiocytosine, 5-methyl-2-thiouracil, 2-thiouracil, 4-thiouracil, 5-methyluracil, uracil-5-oxyacetic acid methylester, uracil-5-oxyacetic acid (v), 5-methyl-2-thiouracil, 3-(3-amino-3-N-2-carboxypropyl) uracil, (acp3)w, and 2,6-diaminopurine.

The antisense oligonucleotide can also comprise at least one modified sugar moiety selected from the group, including but not limited to, arabinose, 2-fluoroarabinose, xylulose, and hexose.

In yet another embodiment, the antisense oligonucleotide will comprise at least one modified phosphate backbone selected from the group consisting of a phosphorothioate, a phosphorodithioate, a phosphoramidothioate, a phosphoramidate, a phosphordiamidate, a methylphosphonate, an alkyl phosphotriester, and a formacetal or analog thereof.

In yet another embodiment, the antisense oligonucleotide is an α-anomeric oligonucleotide. An α-anomeric oligonucleotide forms specific double-stranded hybrids with complementary RNA in which, contrary to the usual β-units, the strands run parallel to each other (Gautier *et al.,* 1987, Nucl. Acids Res. *15*:6625-6641). The oligonucleotide is a 2'-O-methylribonucleotide (Inoue *et al.,* 1987, Nucl. Acids Res. *15*:6131-6148), or a chimeric RNADNA analogue (Inoue *et al.,* 1987, FEBS Lett. *215*:327-330). Alternatively, double stranded RNA can be used to disrupt the expression and function of a targeted NHP.

Oligonucleotides of the invention can be synthesized by standard methods known in the art, e.g. by use of an automated DNA synthesizer (such as are commercially available from Biosearch, Applied Biosystems, etc.). As examples, phosphorothioate oligonucleotides can be synthesized by the method of Stein *et al.* (1988, Nucl. Acids Res. *16*:3209), and methylphosphonate oligonucleotides can be prepared by use of controlled pore glass polymer supports (Sarin *et al*., 1988, Proc. Natl. Acad. Sci. U.S.A. *85*:7448-7451), etc.

Low stringency conditions are well known to those of skill in the art, and will vary predictably depending on the specific organisms from which the library and the labeled sequences are derived. For guidance regarding such conditions see, for example, Sambrook *et al.,* 1989, Molecular Cloning, A Laboratory Manual (and periodic updates thereof), Cold Springs Harbor Press, N.Y.; and Ausubel et *al.,* 1989, Current Protocols in Molecular Biology, Green Publishing Associates and.Wiley Interscience, N.Y.

Alternatively, suitably labeled NHP nucleotide probes can be used to screen a human genomic library using appropriately stringent conditions or by PCR. The identification and characterization of human genomic clones is helpful for identifying polymorphisms (including, but not limited to, nucleotide repeats, microsatellite alleles, single nucleotide polymorphisms, or coding single nucleotide polymorphisms), determining the genomic structure of a given locus/allele, and designing diagnostic tests. For example, sequences derived from regions adjacent to the intron/exon boundaries of the human gene can be used to design primers for use in amplification assays to detect mutations within the exons, introns, splice sites (e.g., splice acceptor and/or donor sites), etc., that can be used in diagnostics and pharmacogenomics.

For example, the present sequences can be used in restriction fragment length polymorphism (RFLP) analysis to identify specific individuals. In this technique, an individual's genomic DNA is digested with one or more restriction enzymes, and probed on a Southern blot to yield unique bands for identification (as generally described in U.S. Pat. No. 5,272,057, incorporated herein by reference). In addition, the sequences of the present invention can be used to provide polynucleotide reagents, e.g., PCR primers, targeted to specific loci in the human genome, which can enhance the reliability of DNA-based forensic identifications by, for example, providing another "identification marker" (i.e., another DNA sequence that is unique to a particular individual). Actual base sequence information can be used for identification as an accurate alternative to patterns formed by restriction enzyme generated fragments

Further, a NHP homolog can be isolated from nucleic acid from an organism of interest by performing PCR using two degenerate or "wobble" oligonucleotide primer pools designed on the basis of amino acid sequences within the NHP products disclosed herein. The template for the reaction may be total RNA, mRNA, and/or cDNA obtained by reverse transcription of mRNA prepared from human or non-human cell lines or tissue known or suspected to express an allele of a NHP gene. The PCR product can be subcloned and sequenced to ensure that the amplified sequences represent the sequence of the desired NHP gene. The PCR fragment can then be used to isolate a full length cDNA clone by a variety of methods. For example, the amplified fragment can be labeled and used to screen a cDNA library, such as a bacteriophage cDNA library. Alternatively, the labeled fragment can be used to isolate genomic clones via the screening of a genomic library.

PCR technology can also be used to isolate full length cDNA sequences. For example, RNA can be isolated, following standard procedures, from an appropriate cellular or tissue source (i.e., one known, or suspected, to express a NHP gene, such as, for example, testis tissue). A reverse transcription (RT) reaction can be performed on the RNA using an oligonucleotide primer specific for the most 5' end of the amplified fragment for the priming of first strand synthesis. The resulting RNA/DNA hybrid may then be "tailed" using a standard terminal transferase reaction, the hybrid may be digested with RNase H, and second strand synthesis may then be primed with a complementary primer. Thus, cDNA sequences upstream of the amplified fragment can be isolated. For a review of cloning strategies that can be used, see *e.g***.,** *Sambrook et al.,* 1989, *supra.*

A cDNA encoding a mutant NHP sequence can be isolated, for example, by using PCR. In this case, the first cDNA strand may be synthesized by hybridizing an oligo-dT oligonucleotide to mRNA isolated from tissue known, or suspected, to be expressed in an individual putatively carrying a mutant NHP allele, and by extending the new strand with reverse transcriptase. The second strand of the cDNA is then synthesized using an oligonucleotide that hybridizes specifically to the 5' end of the normal sequence Using these two primers, the product is then amplified via PCR, optionally cloned into a suitable vector, and subjected to DNA sequence analysis through methods well known to those of skill in the art. By comparing the DNA sequence of the mutant NHP allele to that of a corresponding normal NHP allele, the mutation(s) responsible for the loss or alteration of function of the mutant NHP gene product can be ascertained.

Alternatively, a genomic library can be constructed using DNA obtained from an individual suspected of, or known to carry, a mutant NHP allele (e.g., a person manifesting a NHP-associated phenotype such as, for example, obesity, high blood pressure, arthritis, asthma, connective tissue disorders, infertility, etc.), or a cDNA library can be constructed using RNA from a tissue known, or suspected, to express a mutant NHP allele. A normal NHP gene, or any suitable fragment thereof, can then be labeled and used as a probe to identify the corresponding mutant NHP allele in such libraries. Clones containing mutant NHP sequences can then be purified and subjected to sequence analysis according to methods well known to those skilled in the art.

Additionally, an expression library can be constructed utilizing cDNA synthesized from, for example, RNA isolated from a tissue known, or suspected, to express a mutant NHP allele in an individual suspected of or known to carry such a mutant allele. In this manner, gene products made by the putatively mutant tissue can be expressed and screened using standard antibody screening techniques in conjunction with antibodies raised against normal NHP product, as described below. (For screening techniques, see, for example, Harlow, E. and Lane, eds., 1988, "Antibodies: A Laboratory Manual°, Cold Spring Harbor Press, Cold Spring Harbor.)

Additionally, screening can be accomplished by screening with labeled NHP fusion proteins, such as, for example, alkaline phosphatase-NHP or NHP-alkaline phosphatase fusion proteins. In cases where a NHF mutation results in an expression product with altered function (e.g., as a result of a missense or a frameshift mutation), polyclonal antibodies to NHP are likely to cross-react with a corresponding mutant NHP expression product. Library clones detected via their reaction with such labeled antibodies can be purified and subjected to sequence analysis according to methods well known in the art.

The invention also encompasses (a) DNA vectors that contain any of the foregoing NHP coding sequences and/or their *complements (i.e., antisense);* (b) DNA expression vectors that contain any of the foregoing NHP coding sequences operatively associated with a regulatory element that directs the expression of the coding sequences (for example, baculo virus as described in U.S. Patent No. 5,869,336); (c) genetically engineered host cells that contain any of the foregoing NHP coding sequences operatively associated with a regulatory element that directs the expression of the coding sequences in the host cell; and (d) genetically engineered host cells that express an endogenous NHP sequence under the control of an exogenously introduced regulatory element (i.e., gene activation) or genetically engineered transcription factor. As used herein, regulatory elements include, but are not limited to, inducible and non-inducible promoters, enhancers, operators and other elements known to those skilled in the art that drive and regulate expression. Such regulatory elements include but are not limited to the cytomegalovirus (hCMV) immediate early gene, regulatable, viral elements (particularly retroviral LTR promoters), the early or late promoters of SV40 adenovirus, the *lac* system, the *trp* system, the *TAC* system, the *TRC* system, the major operator and promoter regions of phage lambda, the control regions of fd coat protein, the promoter for 3-phosphoglycerate kinase (PGK), the promoters of acid phosphatase, and the promoters of the yeast α-mating factors.

The present invention also encompasses antibodies and anti-idiotypic antibodies (including Fab fragments), antagonists and agonists of a NHP, as well as compounds or nucleotide constructs that inhibit expression of a NHP sequence (transcription factor inhibitors, antisense and ribozyme molecules, or open reading frame sequence or regulatory sequence replacement constructs), or promote the expression of a NHP (e.g., expression constructs in which NHP coding sequences are operatively associated with expression control elements such as promoters, promoter/enhancers, etc.).

The NHPs or NHP peptides, NHP fusion proteins, NHP nucleotide sequences, antibodies, antagonists and agonists can be useful for the detection of mutant NHPs or inappropriately expressed NHPs for the diagnosis of disease. The NHPs or NHP peptides, NHP fusion proteins, NHP nucleotide sequences, host cell expression systems, antibodies, antagonists, agonists and genetically engineered cells and animals can be used for screening for drugs (or high throughput screening of combinatorial libraries) effective in the treatment of the symptomatic or phenotypic manifestations of perturbing the normal function of NHP in the body. The use of engineered host cells and/or animals may offer an advantage in that such systems allow not only for the identification of compounds that bind to the endogenous receptor for a NHP, but can also identify compounds that trigger NHP-mediated activities or pathways.

Finally, the NHP products can be used as therapeutics. For example, soluble derivatives such as NHP peptides/domains corresponding to NHP, NHP fusion protein products (especially NHP-Ig fusion proteins, i.e., fusions of a NHP, or a domain of a NHP, to an IgFc), NHP antibodies and anti-idiotypic antibodies (including Fab fragments), antagonists or agonists (including compounds that modulate or act on downstream targets in a NHP-mediated pathway) can be used to directly treat diseases or disorders. For instance, in the administration of an effective amount of soluble NHP, or a NHP-IgFc fusion protein or an anti-idiotypic antibody (or its Fab) that mimics a NHP could activate or effectively antagonize the endogenous NHP receptor. Nucleotide constructs encoding such NHP products can be used to genetically engineer host cells to express such products in vivo; these genetically engineered cells function as "bioreactors" in the body delivering a continuous supply of a NHP, a NHP peptide, or a NHP fusion protein to the body. Nucleotide constructs encoding functional NHP, mutant NHPs, as well as antisense and ribozyme molecules can also be used in "gene therapy" approaches for the modulation of NHP expression. Thus, the invention also encompasses pharmaceutical formulations and methods for treating biological disorders.

Various aspects of the invention are described in greater detail in the subsections below.

### 5.1 THE NHP SEQUENCES

The cDNA sequences and corresponding deduced amino acid sequences of the described NHPs are presented in the Sequence Listing. The NHP nucleotides were obtained from human cDNA libraries using probes and/or primers generated from human genomic sequence. Expression analysis has provided evidence that the described NHP can be expressed a variety of human cells.

Several polymorphisms were identified including an A/G polymorphism at the nucleotide position represented by, for example, position 313 of SEQ ID NO: 1 (which can result in a thr or ala at the region corresponding to amino acid (aa) position 105 of, for example, SEQ ID NO:2), an A/T at nucleotide position 1670 (which can result in an asp or val at aa position 557), an A/G at nucleotide position 1864 (which can result in an asp or asn at aa position 622), and a T/C at nucleotide position 1915 (which can result in an phe or leu at aa position 639).

An additional application of the described novel human polynucleotide sequences is their use in the molecular mutagenesis/evolution of proteins that are at least partially encoded by the described novel sequences using, for example, polynucleotide shuffling or related methodologies. Such approaches are described in U.S. Patents Nos: 5,830,721 and 5,837,458.

NHP gene products can also be expressed in transgenic animals. Animals of any species, including, but not limited to, worms, mice, rats, rabbits, guinea pigs, pigs, micro-pigs, birds, goats, and non-human primates, e.g., baboons, monkeys, and chimpanzees may be used to generate NHP transgenic animals.

Any technique known in the art may be used to introduce a NHP transgene into animals to produce the founder lines of transgenic animals. Such techniques include, but are not limited to pronuclear microinjection (Hoppe, P.C. and Wagner, T.E., 1989, U.S. Pat. No. 4, 873, 191) ; retrovirus mediated gene transfer into germ lines (Van der Putten *et al*., 1985, Proc. Natl. Acad. Sci., USA *82*:6148-6152); gene targeting in embryonic stem cells (Thompson et *al.,* 1989, Cell *56*:313-321); electroporation of embryos (Lo, 1983, Mol Cell. Biol. 3:1803-1814); and sperm-mediated gene transfer (Lavitrano *et al.*, 1989, Cell *57*:717-723); etc. For a review of such techniques, see Gordon, 1989, Transgenic Animals, Intl. Rev. Cytol. *115*:171-229.

The present invention provides for transgenic animals that carry the NHP transgene in all their cells, as well as animals which carry the transgene in some, but not all their cells, i.e., mosaic animals or somatic cell transgenic animals. The transgene may be integrated as a single transgene or in concatamers, e.g., head-to-head tandems or head-to-tail tandems. The transgene may also be selectively introduced into and activated in a particular cell type by following, for example, the teaching of Lasko *et al.* 1992, Proc. Natl. Acad. Sci. USA *89*:6232-6236. The regulatory sequences required for such a cell-type specific activation will depend upon the particular cell type of interest, and will be apparent to those of skill in the art.

When it is desired that a NHP transgene be integrated into the chromosomal site of the endogenous NHP gene, gene targeting is preferred. Briefly, when such a technique is to be utilized, vectors containing some nucleotide sequences homologous to the endogenous NHP gene are designed for the purpose of integrating, via homologous recombination with chromosomal sequences, into and disrupting the function of the nucleotide sequence of the endogenous NHP gene (i.e., "knockout" animals).

The transgene can also be selectively introduced into a particular cell type, thus inactivating the endogenous NHP gene in only that cell type, by following, for example, the teaching of Gu *et al.,* 1994, Science, *265*:103-106. The regulatory sequences required for such a cell-type specific inactivation will depend upon the particular cell type of interest, and will be apparent to those of skill in the art.

Once transgenic animals have been generated, the expression of the recombinant NHP gene may be assayed utilizing standard techniques. Initial screening may be accomplished by Southern blot analysis or PCR techniques to analyze animal tissues to assay whether integration of the transgene has taken place. The level of mRNA expression of the transgene in the tissues of the transgenic animals may also be assessed using techniques which include but are not limited to Northern blot analysis of tissue samples obtained from the animal, *in situ* hybridization analysis, and RT-PCR. Samples of NHP gene-expressing tissue, may also be evaluated immunocytochemically using antibodies specific for the NHP transgene product.

### 5.2 NHPS AND NHP POLYPEPTIDES

The NHPs, NHP polypeptides, NHP peptide fragments, mutated, truncated, or deleted forms of NHP, and/or NHP fusion proteins can be prepared for a variety of uses. These uses include but are not limited to the generation of antibodies, as reagents in diagnostic assays, the identification of other cellular gene products related to a NHP, as reagents in assays for screening for compounds that can be used as pharmaceutical reagents useful in the therapeutic treatment of mental, biological, or medical disorders and disease. The described NHPs share similarity with a variety of proteases, including, but not limited to, disintegrins, ADAMs, and fertilins.

The Sequence Listing discloses the amino acid sequences encoded by the described NHP polynucleotides. The NHPs display initiator methionines in DNA sequence contexts consistent with translation initiation sites, and the ORFs display signal-like sequences which can indicate that the described NHP ORFs are secreted proteins or can be membrane associated.

The NHP amino acid sequences of the invention include the amino acid sequences presented in the Sequence Listing as well as analogues and derivatives thereof. Further, corresponding NHP homologues from other species are encompassed by the invention. In fact, any NHPs encoded by a NHP nucleotide sequence described above are within the scope of the invention, as are any novel polynucleotide sequences encoding all or any - novel- portion of an amino acid sequence presented in the Sequence Listing. The degenerate nature of the genetic code is well known, and, accordingly, each amino acid presented in the Sequence Listing, is generically representative of the well known nucleic acid "triplet" codon, or in many cases codons, that can encode the amino acid. As such, as contemplated herein, the amino acid sequences presented in the Sequence Listing, when taken together with the genetic code (see, for example, Table 4-1 at page 109 of "Molecular Cell Siologym, 1986, J. Darnell *et al.* eds.*,* Scientific American Books, New York, NY) are generically representative of all the various permutations and combinations of nucleic acid sequences that can encode such amino acid sequences.

The invention also encompasses proteins that are functionally equivalent to the NHPs encoded by the presently described nucleotide sequences as judged by any of a number of criteria, including, but not limited to, the ability to bind and cleave a substrate of a NHP, or the ability to effect an identical or complementary downstream pathway, or a change in cellular metabolism (e.g., proteolytic activity, ion flux, tyrosine phosphorylation, etc.). Such functionally equivalent NHP proteins include, but are not limited to, additions or substitutions of amino acid residues within the amino acid sequence encoded by the NHP nucleotide sequences described above, but which result in a silent change, thus producing a functionally equivalent expression product.

Amino acid substitutions can be made on the basis of similarity in polarity, charge, solubility, hydrophobicity, hydrophilicity, and/or the amphipathic nature of the residues involved. For example, nonpolar (hydrophobic) amino acids include alanine, leucine, isoleucine, valine, proline, phenylalanine, tryptophan, and methionine; Polar neutral amino acids include glycine, serine, threonine, cysteine, tyrosine, asparagine, and glutamine; positively charged (basic) amino acids include arginine, lysine, and histidine; and negatively charged (acidic) amino acids include aspartic acid and glutamic acid.

A variety of host-expression vector systems can be used to express the NHP nucleotide sequences of the invention. Where, as in the present instance, a NHP peptide or NHP polypeptide is thought to be a soluble or secreted molecule, the peptide or polypeptide can be recovered from the culture media. Such expression systems also encompass engineered host cells that express NHP, or functional equivalent, *in situ.* Purification or enrichment of a NHP from such expression systems can be accomplished using appropriate detergents and lipid micelles and methods well known to those skilled in the art. However, such engineered host cells themselves may be used in situations where it is important not only to retain the structural and functional characteristics of a NHP, but to assess biological activity, *e.g*., in drug screening assays.

The expression systems that may be used for purposes of the invention include, but are not limited to,microorganisms such as bacteria *(e.g., E. coli, B. subtilis)* transformed with recombinant bacteriophage DNA, plasmid DNA or cosmid DNA expression vectors containing NHP nucleotide sequences; yeast *(e.g., Saccharomyces, Pichia)* transformed with recombinant yeast expression vectors containing NHP encoding nucleotide sequences; insect cell systems infected with recombinant virus expression vectors (e.g., baculovirus) containing NHP sequences; plant cell systems infected with recombinant virus expression vectors *(e.g.,* cauliflower mosaic virus, CaMV; tobacco mosaic virus, TMV) or transformed with recombinant plasmid expression vectors (e.g., Ti plasmid) containing NHP nucleotide sequences; or mammalian cell systems (e.g., COS, CHO, BHK, 293, 3T3) harboring recombinant expression.constructs containing promoters derived from the genome of mammalian cells (e.g., metallothionein promoter) or from mammalian viruses (e.g., the adenovirus late promoter; the vaccinia virus 7.5K promoter).

In bacterial systems, a number of expression vectors may be advantageously selected depending upon the use intended for the NHP product being expressed. For example, when a large quantity of such a protein is to be produced for the generation of pharmaceutical compositions of and/or containing a NHP, or for raising antibodies to a NHP, vectors that direct the expression of high levels of fusion protein products that are readily purified may be desirable. Such vectors include, but are not limited, to the *E. coli* expression vector pUR278 (Ruther *et al.,* 1983, EMBO J. 2:1791), in which a NHP coding sequence may be ligated individually into the vector in frame with the *lacZ* coding region so that a fusion protein is produced; pIN vectors (Inouye & Inouye, 1985, Nucleic Acids Res. *13*:3101-3109; Van Heeke & Schuster, 1989, J. Biol. Chem. *264*:5503-5509); and the like. pGEX vectors (Pharmacia or American Type Culture Collection) can also be used to express foreign polypeptides as fusion proteins with glutathione S-transferase (GST). In general, such fusion proteins are soluble and can easily be purified from lysed cells by adsorption to glutathione-agarose beads followed by elution in the presence of free glutathione. The PGEX vectors are designed to include thrombin or factor Xa protease cleavage sites so that the cloned target expression product can be released from the GST moiety.

In an insect system, Autographa *californica* nuclear polyhedrosis virus (ACNPV) is used as a vector to express foreign polynucleotide sequences. The virus grows in *Spodoptera* frugiperda cells. A NHP coding sequence can be cloned individually into non-essential regions (for example the polyhedrin gene) of the virus and placed under control of an AcNPV promoter (for example the polyhedrin promoter). Successful insertion of NHP coding sequence will result in inactivation of the polyhedrin gene and production of non-occluded recombinant virus *(i.e.,* virus lacking the proteinaceous coat coded for by the polyhedrin gene). These recombinant viruses are then used to infect Spodoptera frugiperda cells in which the inserted sequence is expressed *(e.g.,* see Smith *et al.*, 1983, .T. Virol. 46: 584; U.S. Patent No. 4,215,051).

In mammalian host cells, a number of viral-based expression systems may be utilized. In cases where an adenovirus is used as an expression vector, the NHP nucleotide sequence of interest may be ligated to an adenovirus transcription/translation control complex, e.g., the late promoter and tripartite leader sequence. This chimeric sequence may then be inserted in the adenovirus genome by *in vitro* or *in vivo* recombination. Insertion in a non-essential region of the viral genome (*e.g*., region E1 or E3) will result in a recombinant virus that is viable and capable of expressing a NHP product in infected hosts (*e.g.*, See Logan & Shenk, 1984, Proc. Natl. Acad. Sci. USA 81:3655-3659). Specific initiation signals may also be required for efficient translation of inserted NHP nucleotide sequences. These signals include the ATG initiation codon and adjacent sequences. In cases where an entire NHP gene or cDNA, including its own initiation codon and adjacent sequences, is inserted into the appropriate expression vector, no additional translational control signals may be needed. However, in cases where only a portion of a NHP coding sequence is inserted, exogenous translational control signals, including, perhaps, the ATG initiation codon, must be provided. Furthermore, the initiation codon must be in phase with the reading frame of the desired coding sequence to ensure translation of the entire insert. These exogenous translational control signals and initiation codons can be of a variety of origins, both natural and synthetic. The efficiency of expression may be enhanced by the inclusion of appropriate transcription enhancer elements, transcription terminators, etc. (See Bitter et *al.,* 1987, Methods in Enzymol. 153:516-544).

In addition, a host cell strain may be chosen that modulates the expression of the inserted sequences, or modifies and processes the expression product in the specific fashion desired. Such modifications (e.g., glycosylation) and processing (e.g., cleavage) of protein products may be important for the function of the protein. Different host cells have characteristic and specific mechanisms for the post-translational processing and modification of proteins and expression products. Appropriate cell lines or host systems can be chosen to ensure the correct modification and processing of the foreign protein expressed. To this end, eukaryotic host cells which possess the cellular machinery for proper processing of the primary transcript, glycosylation, and phosphorylation of the expression product may be used. Such mammalian host cells include, but are not limited to, CHO, VERO, BHK, HeLa, COS, MDCK, 293, 3T3, WI38, and in particular, human cell lines.

For long-term, high-yield production of recombinant proteins, stable expression is preferred. For example, cell lines which stably express the NHP sequences described above can be engineered. Rather than using expression vectors which contain viral origins of replication, host cells can be transformed with DNA controlled by appropriate expression control elements (e.g., promoter, enhancer sequences, transcription terminators, polyadenylation sites, etc.), and a selectable marker. Following the introduction of the foreign DNA, engineered cells may be allowed to grow for 1-2 days in an enriched media, and then are switched to a selective media. The selectable marker in the recombinant plasmid confers resistance to the selection and allows cells to stably integrate the plasmid into their chromosomes and grow to form foci which in turn can be cloned and expanded into cell lines. This method may advantageously be used to engineer cell lines which express a NHP product. Such engineered cell lines may be particularly useful in screening and evaluation of compounds that affect the endogenous activity of a NHP product.

A number of selection systems may be used, including but not limited to, the herpes simplex virus thymidine kinase (Wigler, *et al*., 1977, Cell *11*:223), hypoxanthine-guanine phosphoribosyltransferase (Szybalska & Szybalski, 1962, Proc. Natl. Acad. Sci. USA *48*:2026), and adenine phosphoribosyltransferase (Lowy, *et al*., 1980, Cell *22*:817) genes, which can be employed in tk⁻, hgprt⁻ or aprt⁻ cells, respectively. Also, antimetabolite resistance can be used as the basis of selection for the following genes: dhfr, which confers resistance to methotrexate (Wigler, *et al*., 1980, Natl. Acad. Sci. USA *77*:3567; O'Hare, *et al.,* 1981, Proc. Natl. Acad. Sci. USA 78:1527); gpt, which confers resistance to mycophenolic acid (Mulligan & Berg, 1981, Proc. Natl. Acad. Sci. USA 78:2072); neo, which confers resistance to the aminoglycoside G-418 (Colberre-Garapin, et *al.,* 1981, J. Mol. Biol. 150:1); and hygro, which confers resistance to hygromycin (Santerre, *et al.,* 1984, Gene *30*:147).

Alternatively, any fusion protein can be readily purified by utilizing an antibody specific for the fusion protein being expressed. For example, a system described by Janknecht *et al.* allows for the ready purification of non-denatured fusion proteins expressed in human cell lines (Janknecht, et *al.,* 1991, Proc. Natl. Acad. Sci. USA 88:8972-8976). In this system, the sequence of interest is subcloned into a vaccinia recombination plasmid such that the sequence's open reading frame is translationally fused to an amino-terminal tag consisting of six histidine residues. Extracts from cells infected with recombinant vaccinia virus are loaded onto Ni²⁺nitriloacetic acid-agarose columns and histidine-tagged proteins are selectively eluted with imidazole-containang buffers.

Also encompassed by the present invention are fusion proteins that direct the NHP to a target organ and/or facilitate transport across the membrane into the cytosol. Conjugation of NHPs to antibody molecules or their Fab fragments could be used to target cells bearing a particular epitope. Attaching the appropriate signal sequence to the NHP would also transport the NHP to the desired location within the cell. Alternatively targeting of NHP or its nucleic acid sequence might be achieved using liposome or lipid complex based delivery systems. Such technologies are described in "Liposomes:A Practical Approach", New, R.R.C., ed., Oxford University Press, New York and in U.S. Patents Nos. 4,594,595, 5,459,127, 5,948,767 and 6,110,490 and their respective disclosures.

Additionally embodied are novel protein constructs engineered in such a way that they facilitate transport of the NHP to the target site or desired organ, where they cross the cell membrane and/or the nucleus where the NHP can exert its functional activity. This goal may be achieved by coupling of the NHP to a cytokine or other ligand that provides targeting specificity, and/or to a protein transducing domain (see generally U.S. applications Ser. No. 60/111,701 and 60/056,713 for examples of such transducing sequences) to facilitate passage across cellular membranes and can optionally be engineered to include nuclear localization.

### 5.3 ANTIBODIES TO NHP PRODUCTS

Antibodies that specifically recognize one or more epitopes of a NHP, or epitopes of conserved variants of a NHP, or peptide fragments of a NHP are also encompassed by the invention. Such antibodies include, but are not limited to polyclonal antibodies, monoclonal antibodies (mAbs), humanized or chimeric antibodies, single chain antibodies, Fab fragments, F(ab')₂ fragments, fragments produced by a Fab expression library, anti-idiotypic (anti-Id) antibodies, and epitope-binding fragments of any of the above.

The antibodies of the invention may be used, for example, in the detection of a NHP in a biological sample and may, therefore, be utilized as part of a diagnostic or prognostic technique whereby patients may be tested for abnormal amounts of NHP. Such antibodies may also be utilized in conjunction with, for example, compound screening schemes for the evaluation of the effect of test compounds on expression and/or activity of a NHP expression product. Additionally, such antibodies can be used in conjunction with gene therapy to, for example, evaluate the normal and/or engineered NHP-expressing cells prior to their introduction into the patient. Such antibodies may additionally be used as a method for the inhibition of abnormal NHP activity. Thus, such antibodies may, therefore, be utilized as part of treatment methods.

For the production of antibodies, various host animals may be immunized by injection with a NHP, an NHP peptide (e.g., one corresponding to a functional domain of a NHP), truncated NHP polypeptides (NHP in which one or more domains have been deleted), functional equivalents of a NHP, or mutated variants of a NHP. Such host animals may include,but are not limited to, pigs, rabbits, mice, goats, and rats, to name but a few. Various adjuvants may be used to increase the immunological response, depending on the host species, including but not limited to, Freund's adjuvant (complete and incomplete), mineral salts such as aluminum hydroxide or aluminum phosphate, chitosan, surface active substances such as lysolecithin, pluronic polyols, polyanions, peptides, oil emulsions, and potentially useful human adjuvants such as BCG (bacille Calmette-Guerin) and Corynebacterium *parvum.* Alternatively, the immune response could be enhanced by combination and or coupling with molecules such as keyhole limpet hemocyanin, tetanus toxoid, diphtheria toxoid, ovalbumin, cholera toxin or fragments thereof. Polyclonal antibodies are heterogeneous populations of antibody molecules derived from the sera of the immunized animals.

Monoclonal antibodies, which are homogeneous populations of antibodies to a particular antigen, can be obtained by any technique which provides for the production of antibody molecules by continuous cell lines in culture. These include, but are not limited to, the hybridoma technique of Kohler and Milstein, (1975, Nature *256*:495-497; and U.S. Patent No. 4,376,110), the human B-cell hybridoma technique (Kosbor *et al.,* 1983, Immunology Today *4*:72; 1983, Proc. Natl. Acad. Sci. USA 80:2026-2030), and the EBV-hybridoma technique (Cole et *al.,* 1985, Monoclonal Antibodies And Cancer Therapy, Alan R. Liss, Inc., pp. 77-96). Such antibodies may be of any immunoglobulin class including IgG, IgM, IgE, IgA, IgD and any subclass thereof. The hybridoma producing the mAb of this invention may be cultivated in vitro or in vivo. Production of high titers of mAbs in vivo makes this the presently preferred method of production.

In addition, techniques developed for the production of "chimeric antibodies" (Morrison *et al.*, 1984, Proc. Natl. Acad. *Sci., 81*:6851-6855; Neuberger *et al.,* 1984, Nature, *312*:604-608; Takeda *et al.,* 1985, Nature, *314*:452-454) by splicing the genes from a mouse antibody molecule of appropriate antigen specificity together with genes from a human antibody molecule of appropriate biological activity can be used (see U.S. Patents Nos. 5,877,397 and 6,075,181. A chimeric antibody is a molecule in which different portions are derived from different animal species, such as those having a variable region derived from a murine mAb and a human immunoglobulin constant region. Such technologies are described in U.S. Patents Nos. 6,075,181 and 5,877,397 and their respective disclosures. Also encompassed by the present invention is the use of fully humanized monoclonal antibodies as described in US Patent No. 6,150,584 and respective disclosures which are herein incorporated by reference in their entirety.

Alternatively, techniques described for the production of single chain antibodies (U.S. Patent 4,946,778; Bird, 1988, Science *242*:423-426; Huston *et al*., 1988, Proc. Natl. Acad. Sci. USA *85*:5879-5883; and Ward *et al*., 1989, Nature *341*:544-546) can be adapted to produce single chain antibodies against NHP expression products. Single chain antibodies are formed by linking the heavy and light chain fragments of the Fv region via an amino acid bridge, resulting in a single chain polypeptide.

Antibody fragments which recognize specific epitopes may be generated by known techniques. For example, such fragments include, but are not limited to: the F(ab')₂ fragments which can be produced by pepsin digestion of the antibody molecule and the Fab fragments which can be generated by reducing the disulfide bridges of the F(ab')₂ fragments. Alternatively, Fab expression libraries may be constructed (Huse et *al.,* 1989, Science, 246:1275-1281) to allow rapid and easy identification of monoclonal Fab fragments with the desired specificity.

Antibodies to a NHP can, in turn, be utilized to generate anti-idiotype antibodies that "mimic" a given NHP, using techniques well known to those skilled in the art. (See, e.g., Greenspan & Bona, 1993, FASEB J *7(5)*:437-444; and Nissinoff, 1991, J. Immunol. *147*(8):2429-2438). For example, antibodies which bind to a NHP domain and competitively inhibit the binding of NHP to its cognate receptor can be used to generate anti-idiotypes that "mimic" a NHP and, therefore, bind and activate or neutralize a receptor. Such anti-idiotypio antibodies or Fab fragments of such anti-idiotypes can be used in therapeutic regimens involving a NHP signaling pathway.

Additionally, given the high degree of relatedness of mammalian NHPs, the presently described knock-out mice (having never seen NHP, and thus never been tolerized to NEP) have a unique utility, as they can be advantageously applied to the generation of antibodies against the disclosed mammalian NHP (i.e., NHP will be immunogenic in NHP knock-out animals).

## Claims

1. An isolated nucleic acid molecule comprising a nucleotide sequence encoding an amino acid sequence drawn from the group consisting of SEQ ID NOS: 2, 4, 6, and 8.

2. An isolated nucleic acid molecule comprising a nucleotide sequence that:
(a) encodes the amino acid sequence shown in SEQ ID NO: 6; and
(b) hybridizes under stringent conditions to the nucleotide sequence of SEQ ID NO: 5 or the complement thereof.

3. An isolated nucleic acid molecule encoding SEQ ID NO:2.

4. An isolated nucleic acid molecule encoding SEQ ID NO:4.

5. A polypeptide encoded by a nucleic acid as claimed in any one of claims 1 to 4.

6. An antibody that specifically recognises one or more epitopes of a polypeptide as claimed in claim 5, or an epitope-binding fragment or a derivative of said antibody, for example, a polyclonal or monoclonal antibody, a single chain antibody, a Fab fragment, a F(ab')2, a fragment produced by a Fab expression library, or an anti-idiotypic (anti-Id) antibody.

7. A genetically engineered host cell that comprises a nucleic acid as claimed in any one of claims 1 to 4 operatively associated with a regulatory element that directs the expression of the nucleic acid in the host cell.

8. A non-human transgenic animal that expresses a nucleic acid as claimed in any one of claims 1 to 4, or that is a "knock-out" animal that does not express a functional polypeptide as claimed in claim 5.

9. Use of an isolated nucleic acid as claimed in any one of claims 1 to 4, a polypeptide as claimed in claim 5, an antibody as claimed in claim 6, a genetically engineered host cell as claimed in claim 7, or a non-human animal as claimed in claim 8, in a method for screening for drugs effective in the treatment of the symptomatic or for the ability to modulate the phenotypic manifestations of perturbing the normal function of an isolated nucleic acid as claimed in any one of claims 1 to 4 or a polypeptide as claimed in claim 5.

## Patentansprüche

1. Ein isoliertes Nukleinsäuremolekül umfassend eine Nukleotidsequenz, die für eine Aminosäuresequenz kodiert, ausgewählt aus der Gruppe bestehend aus SEQ ID NOs: 2, 4, 6 und 8.

2. Isoliertes Nukleinsäuremolekül umfassend eine Nukleotidsequenz, die:
(a) für die Aminosäuresequenz kodiert, die in SEQ ID NO: 6 gezeigt ist; und
(b) unter stringenten Bedingungen mit der Nukleotidsequenz von SEQ ID NO: 5 oder dem Komplement davon hybridisiert.

3. Isoliertes Nukleinsäuremolekül, das für SEQ ID NO: 2 kodiert.

4. Isoliertes Nukleinsäuremolekül, das für SEQ ID NO: 4 kodiert.

5. Polypeptid, das durch eine wie in einem der Ansprüche 1-4 beanspruchte Nukleinsäure kodiert wird.

6. Antikörper, der spezifisch ein oder mehrere Epitope eines Polypeptids wie in Anspruch 5 beansprucht erkennt oder ein Epitop-bindendes Fragment oder ein Derivat des Antikörpers, zum Beispiel ein polyklonaler oder monoklonaler Antikörper, ein einsträngiger Antikörper, ein Fab-Fragment, ein F(ab')2, ein Fragment, hergestellt mittels einer Fab-Expressions-Bibliothek, oder ein anti-idiotypischer (anti-Id) Antikörper.

7. Eine gentechnisch manipulierte Wirtszelle, umfassend eine Nukleinsäure wie in einem der Ansprüche 1-4 beansprucht, operativ assoziiert mit einem regulatorischen Element, welches die Expression der Nukleinsäure in der Wirtszelle steuert.

8. Ein nicht-humanes, transgenes Tier, welches eine Nukleinsäure wie in einem der Ansprüche 1-4 beansprucht exprimiert oder welches ein "knock-out"-Tier ist, das kein funktionelles Polypeptid wie in Anspruch 5 beansprucht exprimiert.

9. Verwendung einer isolierten Nukleinsäure wie in einem der Ansprüche 1 bis 4 beansprucht, eines Polypeptids wie in Anspruch 5 beansprucht, eines Antikörpers wie in Anspruch 6 beansprucht, einer genetisch manipulierten Wirtszelle wie in Anspruch 7 beansprucht, oder eines nicht-humanen Tieres wie in Anspruch 8 beansprucht, in einem Screening-Verfahren nach Wirkstoffen, die bei der Behandlung des Symptomatischen wirksam sind oder nach der Fähigkeit die phenotypischen Manifestationen von Störungen der Normalfunktion einer isolierten Nukleinsäure wie in einem der Ansprüche 1 bis 4 beansprucht oder eines Polypeptids wie in Anspruch 5 beansprucht zu modulieren.

## Revendications

1. Molécule d'acide nucléique isolé comprenant une séquence de nucléotides codant pour une séquence d'acides aminés tirée du groupe constitué par les SEQ ID n° 2, 4, 6 et 8.

2. Molécule d'acide nucléique isolé comprenant une séquence de nucléotides qui :
(a) code pour la séquence d'acides aminés présentée dans SEQ ID n° 6 ; et
(b) s'hybride dans des conditions stringentes à la séquence de nucléotides de SEQ ID n° 5 ou à son complément.

3. Molécule d'acide nucléique isolé codant pour SEQ ID n° 2.

4. Molécule d'acide nucléique isolé codant pour SEQ ID n° 4.

5. Polypeptide codé par un acide nucléique selon l'une quelconque des revendications 1 à 4.

6. Anticorps qui reconnaît spécifiquement un ou plusieurs épitopes d'un polypeptide selon la revendication 5, ou un fragment se liant à l'épitope ou un dérivé dudit anticorps, par exemple un anticorps polyclonal ou monoclonal, un anticorps simple chaîne, un fragment Fab, un fragment F(ab')2, un fragment produit par une banque d'expression de Fab, ou un anticorps anti-idiotypique (anti-Id).

7. Cellule hôte manipulée génétiquement qui comprend un acide nucléique selon l'une quelconque des revendications 1 à 4 en association fonctionnelle avec un élément régulateur qui dirige l'expression de l'acide nucléique dans la cellule hôte.

8. Animal transgénique non humain qui exprime un acide nucléique selon l'une quelconque des revendications 1 à 4, ou qui est un animal « knock-out » qui n'exprime pas de polypeptide fonctionnel selon la revendication 5.

9. Utilisation d'un acide nucléique isolé selon l'une quelconque des revendications 1 à 4, d'un polypeptide selon la revendication 5, d'un anticorps selon la revendication 6, d'une cellule hôte manipulée génétiquement selon la revendication 7 ou d'un animal non humain selon la revendication 8, dans un procédé pour cribler des médicaments efficaces dans le traitement des manifestations symptomatiques ou pour pouvoir moduler les manifestations phénotypiques de la perturbation de la fonction normale d'un acide nucléique isolé selon l'une quelconque des revendications 1 à 4 ou d'un polypeptide selon la revendication 5.


### SEQUENCE LISTING

<110> LEXICON GENETICS INCORPORATED
<120> Novel Human Proteases and Polynucleotides Encoding the Same
<130> LEX-0263-PCT
<150> US 60/244,939
   <151> 2000-11-01
<160> 9
<170> FastSEQ for Windows Version 4.0
<210> 1
   <211> 2781
   <212> DNA
   <213> homo sapiens
<400> 1
   atgccagggg gcgcaggcgc cgcccggctc tgcttgctgg cgtttgccct gcagcccctc      60
   cggccgcggg cggcgcggga gcctggatgg acaagaggaa gtgaggaagg cagccccaag     120
   ctgcagcatg aacttatcat acctcagtgg aagacttcag aaagccccgt gagagaaaag     180
   catccactca aagctgagct cagggtaatg gctgaggggc gagaactgat cctggacctg     240
   gagaagaatg agcaactttt tgctccttcc tacacagaaa cccattatac ttcaagtggt     300
   aaccctcaaa ccaccacacg gaaattggag gatcactgct tttaccacgg cacggtgagg     360
   gagacagaac tgtccagcgt cacgctcagc acttgccgag gaattagagg actgattacg     420
   gtgagcagca acctcagcta cgtcatcgag cccctccctg acagcaaggg ccaacacctt     480
   atttacagat ctgaacatct caagccgccc ccgggaaact gtgggttcga gcactccaag     540
   cccaccacca gggactgggc tcttcagttt acacaacaga ccaagaagcg acctcgcagg     600
   atgaaaaggg aagatttaaa ctccatgaag tatgtggagc tttacctcgt ggctgattat     660
   ttagagtttc agaagaatcg acgagaccag gacgccacca aacacaagct catagagatc     720
   gccaactatg ttgataagtt ttaccgatcc ttgaacatcc ggattgctct cgtgggcttg     780
   gaagtgtgga cccacgggaa catgtgtgaa gtttcagaga atccatattc taccctctgg     840
   tcctttctca gttggaggcg caagctgctt gcccagaagt accatgacaa cgcccaatta     900
   atcacgggca tgtccttcca cggcaccacc atcggcctgg cccccctcat ggccatgtgc    960
   tctgtgtacc agtctggagg agtcaacatg gaccactccg agaatgccat tggcgtggct    1020
   gccaccatgg cccacgagat gggccacaac tttggcatga cccatgattc tgcagattgc    1080
   tgctcggcca gtgcggctga tggtgggtgc atcatggcag ctgccactgg gcaccccttt   1140
   cccaaagtgt tcaatggatg caacaggagg gagctggaca ggtatctgca gtcaggtggt    1200
   ggaatgtgtc tctccaacat gccagacacc aggatgttgt atggaggccg gaggtgtggg    1260
   aacgggtatc tggaagatgg ggaagagtgt gactgtggag aagaagagga atgtaacaac    1320
   ccctgctgca atgcctctaa ttgtaccctg aggccggggg cggagtgtgc tcacggctcc    1380
   tgctgccacc agtgtaagct gttggctcct gggaccctgt gccgcgagca ggccaggcag    1440
   tgtgacctcc cggagttctg tacgggcaag tctccccact gccctaccaa cttctaccag    1500
   atggatggta ccccctgtga gggcggccag gcctactgct acaacggcat gtgcctcacc    1560
   taccaggagc agtgccagca gctgtgggga cccggagccc gacctgcccc tgacctctgc    1620
   ttcgagaagg tgaatgtggc aggagacacc tttggaaact gtggaaagga catgaatggt    1680
   gaacacagga agtgcaacat gagagatgcg aagtgtggga agatccagtg tcagagctct    1740
   gaggcccggc ccctggagtc caacgcggtg cccattgaca ccactatcat catgaatggg    1800
   aggcagatcc agtgccgggg cacccacgtc taccgaggtc ctgaggagga gggtgacatg    1860
   ctggacccag ggctggtgat gactggaacc aagtgtggct acaaccatat ttgctttgag    1920
   gggcagtgca ggaacacctc cttctttgaa actgaaggct gtgggaagaa gtgcaatggc    1980
   catggggtct gtaacaacaa ccagaactgc cactgcctgc cgggctgggc cccgcccttc    2040
   tgcaacacac cgggccacgg gggcagtatc gacagtgggc ctatgccccc tgagagtgtg    2100
   ggtcctgtgg tagctggagt gttggtggcc atcttggtgc tggcggtcct catgctgatg    2160
   tactactgct gcagacagaa caacaaacta ggccaactca agccctcagc tctcccttcc    2220
   aagctgaggc aacagttcag ttgtcccttc agggtttctc agaacagcgg gactggtcat    2280
   gccaacccaa ctttcaagct gcagacgccc cagggcaagc gaaaggtgtt ccttgacttg    2340
   tgcgtacagg tgatcaacac tccggaaatc ctgcggaagc cctcccagcc tcctccccgg    2400
   ccccctccag attatctgcg tggtgggtcc ccacctgcac cactgccagc tcacctgagc    2460
   agggctgcta ggaactcccc agggcccggg tctcaaatag agaggacgga gtcgtccagg    2520
   aggcctcctc caagccggcc aattcccccc gcaccaaatt gcatcgtttc ccaggacttc    2580
   tccaggcctc ggccgcccca gaaggcactc ccggcaaacc cagtgccagg ccgcaggagc    2640
   ctccccaggc caggaggtgc atccccactg cggccccctg gtgctggccc tcagcagtcc    2700
   cggcctctgg cagcacttgc cccaaagagg gtatggaaga cttgcaattt gaaaactggg    2760
   gaccagttcc aaagtcagta a                                              2781
<210> 2
   <211> 926
   <212> PRT
   <213> homo sapiens
<400> 2
   Met Pro Gly Gly Ala Gly Ala Ala Arg Leu Cys Leu Leu Ala Phe Ala
    1               5                  10                  15
   Leu Gln Pro Leu Arg Pro Arg Ala Ala Arg Glu Pro Gly Trp Thr Arg
               20                  25                  30
   Gly Ser Glu Glu Gly Ser Pro Lys Leu Gln His Glu Leu Ile Ile Pro
           35                  40                  45
   Gln Trp Lys Thr Ser Glu Ser Pro Val Arg Glu Lys His Pro Leu Lys
       50                  55                  60
   Ala Glu Leu Arg Val Met Ala Glu Gly Arg Glu Leu Ile Leu Asp Leu
   65                  70                  75                  80
   Glu Lys Asn Glu Gln Leu Phe Ala Pro Ser Tyr Thr Glu Thr His Tyr
                   85                  90                  95
   Thr Ser Ser Gly Asn Pro Gln Thr Thr Thr Arg Lys Leu Glu Asp His
               100                 105                 110
   Cys Phe Tyr His Gly Thr Val Arg Glu Thr Glu Leu Ser Ser Val Thr
           115                 120                 125
   Leu Ser Thr Cys Arg Gly Ile Arg Gly Leu Ile Thr Val Ser Ser Asn
       130                 135                 140
   Leu Ser Tyr Val Ile Glu Pro Leu Pro Asp Ser Lys Gly Gln His Leu
   145                 150                 155                 160
   Ile Tyr Arg Ser Glu His Leu Lys Pro Pro Pro Gly Asn Cys Gly Phe
                   165                 170                 175
   Glu His Ser Lys Pro Thr Thr Arg Asp Trp Ala Leu Gln Phe Thr Gln
               180                 185                 190
   Gln Thr Lys Lys Arg Pro Arg Arg Met Lys Arg Glu Asp Leu Asn Ser
           195                 200                 205
   Met Lys Tyr Val Glu Leu Tyr Leu Val Ala Asp Tyr Leu Glu Phe Gln
       210                 215                 220
   Lys Asn Arg Arg Asp Gln Asp Ala Thr Lys His Lys Leu Ile Glu Ile
   225                 230                 235                 240
   Ala Asn Tyr Val Asp Lys Phe Tyr Arg Ser Leu Asn Ile Arg Ile Ala
                   245                 250                 255
   Leu Val Gly Leu Glu Val Trp Thr His Gly Asn Met Cys Glu Val Ser
               260                 265                 270
   Glu Asn Pro Tyr Ser Thr Leu Trp Ser Phe Leu Ser Trp Arg Arg Lys
           275                 280                 285
   Leu Leu Ala Gln Lys Tyr His Asp Asn Ala Gln Leu Ile Thr Gly Met
       290                 295                 300
   Ser Phe His Gly Thr Thr Ile Gly Leu Ala Pro Leu Met Ala Met Cys
   305                 310                 315                 320
   Ser Val Tyr Gln Ser Gly Gly Val Asn Met Asp His Ser Glu Asn Ala
                   325                 330                 335
   Ile Gly Val Ala Ala Thr Met Ala His Glu Met Gly His Asn Phe Gly
               340                 345                 350
   Met Thr His Asp Ser Ala Asp Cys Cys Ser Ala Ser Ala Ala Asp Gly
           355                 360                 365
   Gly Cys Ile Met Ala Ala Ala Thr Gly His Pro Phe Pro Lys Val Phe
       370                 375                 380
   Asn Gly Cys Asn Arg Arg Glu Leu Asp Arg Tyr Leu Gln Ser Gly Gly
   385                 390                 395                 400
   Gly Met Cys Leu Ser Asn Met Pro Asp Thr Arg Met Leu Tyr Gly Gly
                   405                 410                 415
   Arg Arg Cys Gly Asn Gly Tyr Leu Glu Asp Gly Glu Glu Cys Asp Cys
               420                 425                 430
   Gly Glu Glu Glu Glu Cys Asn Asn Pro Cys Cys Asn Ala Ser Asn Cys
           435                 440                 445
   Thr Leu Arg Pro Gly Ala Glu Cys Ala His Gly Ser Cys Cys His Gln
       450                 455                 460
   Cys Lys Leu Leu Ala Pro Gly Thr Leu Cys Arg Glu Gln Ala Arg Gln
   465                 470                 475                 480
   Cys Asp Leu Pro Glu Phe Cys Thr Gly Lys Ser Pro His Cys Pro Thr
                   485                 490                 495
   Asn Phe Tyr Gln Met Asp Gly Thr Pro Cys Glu Gly Gly Gln Ala Tyr
               500                 505                 510
   Cys Tyr Asn Gly Met Cys Leu Thr Tyr Gln Glu Gln Cys Gln Gln Leu
           515                 520                 525
   Trp Gly Pro Gly Ala Arg Pro Ala Pro Asp Leu Cys Phe Glu Lys Val
       530                 535                 540
   Asn Val Ala Gly Asp Thr Phe Gly Asn Cys Gly Lys Asp Met Asn Gly
   545                 550                 555                 560
   Glu His Arg Lys Cys Asn Met Arg Asp Ala Lys Cys Gly Lys Ile Gln
                   565                 570                 575
   Cys Gln Ser Ser Glu Ala Arg Pro Leu Glu Ser Asn Ala Val Pro Ile
               580                 585                 590
   Asp Thr Thr Ile Ile Met Asn Gly Arg Gln Ile Gln Cys Arg Gly Thr
           595                 600                 605
   His Val Tyr Arg Gly Pro Glu Glu Glu Gly Asp Met Leu Asp Pro Gly
       610                 615                 620
   Leu Val Met Thr Gly Thr Lys Cys Gly Tyr Asn His Ile Cys Phe Glu
   625                 630                 635                 640
   Gly Gln Cys Arg Asn Thr Ser Phe Phe Glu Thr Glu Gly Cys Gly Lys
                   645                 650                 655
   Lys Cys Asn Gly His Gly Val Cys Asn Asn Asn Gln Asn Cys His Cys
               660                 665                 670
   Leu Pro Gly Trp Ala Pro Pro Phe Cys Asn Thr Pro Gly His Gly Gly
           675                 680                 685
   Ser Ile Asp Ser Gly Pro Met Pro Pro Glu Ser Val Gly Pro Val Val
       690                 695                 700
   Ala Gly Val Leu Val Ala Ile Leu Val Leu Ala Val Leu Met Leu Met
   705                 710                 715                 720
   Tyr Tyr Cys Cys Arg Gln Asn Asn Lys Leu Gly Gln Leu Lys Pro Ser
                   725                 730                 735
   Ala Leu Pro Ser Lys Leu Arg Gln Gln Phe Ser Cys Pro Phe Arg Val
               740                 745                 750
   Ser Gln Asn Ser Gly Thr Gly His Ala Asn Pro Thr Phe Lys Leu Gln
           755                 760                 765
   Thr Pro Gln Gly Lys Arg Lys Val Phe Leu Asp Leu Cys Val Gln Val
       770                 775                 780
   Ile Asn Thr Pro Glu Ile Leu Arg Lys Pro Ser Gln Pro Pro Pro Arg
   785                 790                 795                 800
   Pro Pro Pro Asp Tyr Leu Arg Gly Gly Ser Pro Pro Ala Pro Leu Pro
                   805                 810                 815
   Ala His Leu Ser Arg Ala Ala Arg Asn Ser Pro Gly Pro Gly Ser Gln
               820                 825                 830
   Ile Glu Arg Thr Glu Ser Ser Arg Arg Pro Pro Pro Ser Arg Pro Ile
           835                 840                 845
   Pro Pro Ala Pro Asn Cys Ile Val Ser Gln Asp Phe Ser Arg Pro Arg
       850                 855                 860
   Pro Pro Gln Lys Ala Leu Pro Ala Asn Pro Val Pro Gly Arg Arg Ser
   865                 870                 875                 880
   Leu Pro Arg Pro Gly Gly Ala Ser Pro Leu Arg Pro Pro Gly Ala Gly
                   885                 890                 895
   Pro Gln Gln Ser Arg Pro Leu Ala Ala Leu Ala Pro Lys Arg Val Trp
               900                 905                 910
   Lys Thr Cys Asn Leu Lys Thr Gly Asp Gln Phe Gln Ser Gln
           915                 920                 925
<210> 3
   <211> 2757
   <212> DNA
   <213> homo sapiens
<400> 3
   atgccagggg gcgcaggcgc cgcccggctc tgcttgctgg cgtttgccct gcagcccctc     60
   cggccgcggg cggcgcggga gcctggatgg acaagaggaa gtgaggaagg cagccccaag    120
   ctgcagcatg aacttatcat acctcagtgg aagacttcag aaagccccgt gagagaaaag    180
   catccactca aagctgagct cagggtaatg gctgaggggc gagaactgat cctggacctg    240
   gagaagaatg agcaactttt tgctccttcc tacacagaaa cccattatac ttcaagtggt    300
   aaccctcaaa ccaccacacg gaaattggag gatcactgct tttaccacgg cacggtgagg    360
   gagacagaac tgtccagcgt cacgctcagc acttgccgag gaattagagg actgattacg    420
   gtgagcagca acctcagcta cgtcatcgag cccctccctg acagcaaggg ccaacacctt    480
   atttacagat ctgaacatct caagccgccc ccgggaaact gtgggttcga gcactccaag    540
   cccaccacca gggactgggc tcttcagttt acacaacaga ccaagaagcg acctcgcagg    600
   atgaaaaggg aagatttaaa ctccatgaag tatgtggagc tttacctcgt ggctgattat    660
   ttagagtttc agaagaatcg acgagaccag gacgccacca aacacaagct catagagatc    720
   gccaactatg ttgataagtt ttaccgatcc ttgaacatcc ggattgctct cgtgggcttg    780
   gaagtgtgga cccacgggaa catgtgtgaa gtttcagaga atccatattc taccctctgg    840
   tcctttctca gttggaggcg caagctgctt gcccagaagt accatgacaa cgcccaatta    900
   atcacgggca tgtccttcca cggcaccacc atcggcctgg cccccctcat ggccatgtgc    960
   tctgtgtacc agtctggagg agtcaacatg gaccactccg agaatgccat tggcgtggct   1020
   gccaccatgg cccacgagat gggccacaac tttggcatga cccatgattc tgcagattgc   1080
   tgctcggcca gtgcggctga tggtgggtgc atcatggcag ctgccactgg gcaccccttt   1140
   cccaaagtgt tcaatggatg caacaggagg gagctggaca ggtatctgca gtcaggtggt   1200
   ggaatgtgtc tctccaacat gccagacacc aggatgttgt atggaggccg gaggtgtggg   1260
   aacgggtatc tggaagatgg ggaagagtgt gactgtggag aagaagagga atgtaacaac   1320
   ccctgctgca atgcctctaa ttgtaccctg aggccggggg cggagtgtgc tcacggctcc   1380
   tgctgccacc agtgtaagct gttggctcct gggaccctgt gccgcgagca ggccaggcag   1440
   tgtgacctcc cggagttctg tacgggcaag tctccccact gccctaccaa cttctaccag   1500
   atggatggta ccccctgtga gggcggccag gcctactgct acaacggcat gtgcctcacc   1560
   taccaggagc agtgccagca gctgtgggga cccggagccc gacctgcccc tgacctctgc   1620
   ttcgagaagg tgaatgtggc aggagacacc tttggaaact gtggaaagga catgaatggt   1680
   gaacacagga agtgcaacat gagagatgcg aagtgtggga agatccagtg tcagagctct   1740
   gaggcccggc ccctggagtc caacgcggtg cccattgaca ccactatcat catgaatggg   1800
   aggcagatcc agtgccgggg cacccacgtc taccgaggtc ctgaggagga gggtgacatg   1860
   ctggacccag ggctggtgat gactggaacc aagtgtggct acaaccatat ttgctttgag   1920
   gggcagtgca ggaacacctc cttctttgaa actgaaggct gtgggaagaa gtgcaatggc   1980
   catggggtct gtaacaacaa ccagaactgc cactgcctgc cgggctgggc cccgcccttc    2040
   tgcaacacac cgggccacgg gggcagtatc gacagtgggc ctatgccccc tgagagtgtg   2100
   ggtcctgtgg tagctggagt gttggtggcc atcttggtgc tggcggtcct catgctgatg   2160
   tactactgct gcagacagaa caacaaacta ggccaactca agccctcagc tctcccttcc   2220
   aagctgaggc aacagttcag ttgtcccttc agggtttctc agaacagcgg gactggtcat   2280
   gccaacccaa ctttcaagct gcagacgccc cagggcaagc gaaaggtgat caacactccg   2340
   gaaatcctgc ggaagccctc ccagcctcct ccccggcccc ctccagatta tctgcgtggt   2400
   gggtccccac ctgcaccact gccagctcac ctgagcaggg ctgctaggaa ctccccaggg   2460
   cccgggtctc aaatagagag gacggagtcg tccaggaggc ctcctccaag ccggccaatt   2520
   ccccccgcac caaattgcat cgtttcccag gacttctcca ggcctcggcc gccccagaag   2580
   gcactcccgg caaacccagt gccaggccgc aggagcctcc ccaggccagg aggtgcatcc   2640
   ccactgcggc cccctggtgc tggccctcag cagtcccggc ctctggcagc acttgcccca   2700
   aagagggtat ggaagacttg caatttgaaa actggggacc agttccaaag tcagtaa      2757
<210> 4
   <211> 918
   <212> PRT
   <213> homo sapiens
<400> 4
   Met Pro Gly Gly Ala Gly Ala Ala Arg Leu Cys Leu Leu Ala Phe Ala
   1 5 10 15
   Leu Gln Pro Leu Arg Pro Arg Ala Ala Arg Glu Pro Gly Trp Thr Arg
   20 25 30
   Gly Ser Glu Glu Gly Ser Pro Lys Leu Gln His Glu Leu Ile Ile Pro
   35 40 45
   Gln Trp Lys Thr Ser Glu Ser Pro Val Arg Glu Lys His Pro Leu Lys
   50 55 60
   Ala Glu Leu Arg Val Met Ala Glu Gly Arg Glu Leu Ile Leu Asp Leu
   65 70 75 80
   Glu Lys Asn Glu Gln Leu Phe Ala Pro Ser Tyr Thr Glu Thr His Tyr
   85 90 95
   Thr Ser Ser Gly Asn Pro Gln Thr Thr Thr Arg Lys Leu Glu Asp His
   100 105 110
   Cys Phe Tyr His Gly Thr Val Arg Glu Thr Glu Leu Ser Ser Val Thr
   115 120 125
   Leu Ser Thr Cys Arg Gly Ile Arg Gly Leu Ile Thr Val Ser Ser Asn
   130 135 140
   Leu Ser Tyr Val Ile Glu Pro Leu Pro Asp Ser Lys Gly Gln His Leu
   145 150 155 160
   Ile Tyr Arg Ser Glu His Leu Lys Pro Pro Pro Gly Asn Cys Gly Phe
   165 170 175
   Glu His Ser Lys Pro Thr Thr Arg Asp Trp Ala Leu Gln Phe Thr Gln
   180 185 190
   Gln Thr Lys Lys Arg Pro Arg Arg Met Lys Arg Glu Asp Leu Asn Ser
   195 200 205
   Met Lys Tyr Val Glu Leu Tyr Leu Val Ala Asp Tyr Leu Glu Phe Gln
   210 215 220
   Lys Asn Arg Arg Asp Gln Asp Ala Thr Lys His Lys Leu Ile Glu Ile
   225                 230                 235                 240
   Ala Asn Tyr Val Asp Lys Phe Tyr Arg Ser Leu Asn Ile Arg Ile Ala
                   245                 250                 255
   Leu Val Gly Leu Glu Val Trp Thr His Gly Asn Met Cys Glu Val Ser
               260                 265                 270
   Glu Asn Pro Tyr Ser Thr Leu Trp Ser Phe Leu Ser Trp Arg Arg Lys
           275                 280                 285
   Leu Leu Ala Gln Lys Tyr His Asp Asn Ala Gln Leu Ile Thr Gly Met
       290                 295                 300
   Ser Phe His Gly Thr Thr Ile Gly Leu Ala Pro Leu Met Ala Met Cys
   305                 310                 315                 320
   Ser Val Tyr Gln Ser Gly Gly Val Asn Met Asp His Ser Glu Asn Ala
                   325                 330                 335
   Ile Gly Val Ala Ala Thr Met Ala His Glu Met Gly His Asn Phe Gly
               340                 345                 350
   Met Thr His Asp Ser Ala Asp Cys Cys Ser Ala Ser Ala Ala Asp Gly
           355                 360                 365
   Gly Cys Ile Met Ala Ala Ala Thr Gly His Pro Phe Pro Lys Val Phe
       370                 375                 380
   Asn Gly Cys Asn Arg Arg Glu Leu Asp Arg Tyr Leu Gln Ser Gly Gly
   385                 390                 395                 400
   Gly Met Cys Leu Ser Asn Met Pro Asp Thr Arg Met Leu Tyr Gly Gly
                   405                 410                 415
   Arg Arg Cys Gly Asn Gly Tyr Leu Glu Asp Gly Glu Glu Cys Asp Cys
               420                 425                 430
   Gly Glu Glu Glu Glu Cys Asn Asn Pro Cys Cys Asn Ala Ser Asn Cys
           435                 440                 445
   Thr Leu Arg Pro Gly Ala Glu Cys Ala His Gly Ser Cys Cys His Gln
       450                 455                 460
   Cys Lys Leu Leu Ala Pro Gly Thr Leu Cys Arg Glu Gln Ala Arg Gln
   465                 470                 475                 480
   Cys Asp Leu Pro Glu Phe Cys Thr Gly Lys Ser Pro His Cys Pro Thr
                   485                 490                 495
   Asn Phe Tyr Gln Met Asp Gly Thr Pro Cys Glu Gly Gly Gln Ala Tyr
               500                 505                 510
   Cys Tyr Asn Gly Met Cys Leu Thr Tyr Gln Glu Gln Cys Gln Gln Leu
           515                 520                 525
   Trp Gly Pro Gly Ala Arg Pro Ala Pro Asp Leu Cys Phe Glu Lys Val
       530                 535                 540
   Asn Val Ala Gly Asp Thr Phe Gly Asn Cys Gly Lys Asp Met Asn Gly
   545                 550                 555                 560
   Glu His Arg Lys Cys Asn Met Arg Asp Ala Lys Cys Gly Lys Ile Gln
                   565                 570                 575
   Cys Gln Ser Ser Glu Ala Arg Pro Leu Glu Ser Asn Ala Val Pro Ile
               580                 585                 590
   Asp Thr Thr Ile Ile Met Asn Gly Arg Gln Ile Gln Cys Arg Gly Thr
           595                 600                 605
   His Val Tyr Arg Gly Pro Glu Glu Glu Gly Asp Met Leu Asp Pro Gly
       610                 615                 620
   Leu Val Met Thr Gly Thr Lys Cys Gly Tyr Asn His Ile Cys Phe Glu
   625                 630                 635                 640
   Gly Gln Cys Arg Asn Thr Ser Phe Phe Glu Thr Glu Gly Cys Gly Lys
                   645                 650                 655
   Lys Cys Asn Gly His Gly Val Cys Asn Asn Asn Gln Asn Cys His Cys
               660                 665                 670
   Leu Pro Gly Trp Ala Pro Pro Phe Cys Asn Thr Pro Gly His Gly Gly
   675 680 685
   Ser Ile Asp Ser Gly Pro Met Pro Pro Glu Ser Val Gly Pro Val Val
   690 695 700
   Ala Gly Val Leu Val Ala Ile Leu Val Leu Ala Val Leu Met Leu Met
   705 710 715 720
   Tyr Tyr Cys Cys Arg Gln Asn Asn Lys Leu Gly Gln Leu Lys Pro Ser
   725 730 735
   Ala Leu Pro Ser Lys Leu Arg Gln Gln Phe Ser Cys Pro Phe Arg Val
   740 745 750
   Ser Gln Asn Ser Gly Thr Gly His Ala Asn Pro Thr Phe Lys Leu Gln
   755 760 765
   Thr Pro Gln Gly Lys Arg Lys Val Ile Asn Thr Pro Glu Ile Leu Arg
   770 775 780
   Lys Pro Ser Gln Pro Pro Pro Arg Pro Pro Pro Asp Tyr Leu Arg Gly
   785 790 795 800
   Gly Ser Pro Pro Ala Pro Leu Pro Ala His Leu Ser Arg Ala Ala Arg
   805 810 815
   Asn Ser Pro Gly Pro Gly Ser Gln Ile Glu Arg Thr Glu Ser Ser Arg
   820 825 830
   Arg Pro Pro Pro Ser Arg Pro Ile Pro Pro Ala Pro Asn Cys Ile Val
   835 840 845
   Ser Gln Asp Phe Ser Arg Pro Arg Pro Pro Gln Lys Ala Leu Pro Ala
   850 855 860
   Asn Pro Val Pro Gly Arg Arg Ser Leu Pro Arg Pro Gly Gly Ala Ser
   865 870 875 880
   Pro Leu Arg Pro Pro Gly Ala Gly Pro Gln Gln Ser Arg Pro Leu Ala
   885 890 895
   Ala Leu Ala Pro Lys Arg Val Trp Lys Thr Cys Asn Leu Lys Thr Gly
   900 905 910
   Asp Gln Phe Gln Ser Gln
   915
<210> 5
   <211> 2892
   <212> DNA
   <213> homo sapiens
<400> 5
   atgccagggg gcgcaggcgc cgcccggctc tgcttgctgg cgtttgccct gcagcccctc      60
   cggccgcggg cggcgcggga gcctggatgg acaagaggaa gtgaggaagg cagccccaag     120
   ctgcagcatg aacttatcat acctcagtgg aagacttcag aaagccccgt gagagaaaag     180
   catccactca aagctgagct cagggtaatg gctgaggggc gagaactgat cctggacctg     240
   gagaagaatg agcaactttt tgctccttcc tacacagaaa cccattatac ttcaagtggt     300
   aaccctcaaa ccaccacacg gaaattggag gatcactgct tttaccacgg cacggtgagg     360
   gagacagaac tgtccagcgt cacgctcagc acttgccgag gaattagagg actgattacg     420
   gtgagcagca acctcagcta cgtcatcgag cccctccctg acagcaaggg ccaacacctt     480
   atttacagat ctgaacatct caagccgccc ccgggaaact gtgggttcga gcactccaag     540
   cccaccacca gggactgggc tcttcagttt acacaacaga ccaagaagcg acctcgcagg     600
   atgaaaaggg aagatttaaa ctccatgaag tatgtggagc tttacctcgt ggctgattat     660
   ttagagtttc agaagaatcg acgagaccag gacgccacca aacacaagct catagagatc     720
   gccaactatg ttgataagtt ttaccgatcc ttgaacatcc ggattgctct cgtgggcttg     780
   gaagtgtgga cccacgggaa catgtgtgaa gtttcagaga atccatattc taccctctgg     840
   tcctttctca gttggaggcg caagctgctt gcccagaagt accatgacaa cgcccaatta     900
   atcacgggca tgtccttcca cggcaccacc atcggcctgg cccccctcat ggccatgtgc     960
   tctgtgtacc agtctggagg agtcaacatg gaccactccg agaatgccat tggcgtggct    1020
   gccaccatgg cccacgagat gggccacaac tttggcatga cccatgattc tgcagattgc    1080
   tgctcggcca gtgcggctga tggtgggtgc atcatggcag ctgccactgg gcaccccttt    1140
   cccaaagtgt tcaatggatg caacaggagg gagctggaca ggtatctgca gtcaggtggt    1200
   ggaatgtgtc tctccaacat gccagacacc aggatgttgt atggaggccg gaggtgtggg    1260
   aacgggtatc tggaagatgg ggaagagtgt gactgtggag aagaagagga atgtaacaac    1320
   ccctgctgca atgcctctaa ttgtaccctg aggccggggg cggagtgtgc tcacggctcc    1380
   tgctgccacc agtgtaagct gttggctcct gggaccctgt gccgcgagca ggccaggcag    1440
   tgtgacctcc cggagttctg tacgggcaag tctccccact gccctaccaa cttctaccag    1500
   atggatggta ccccctgtga gggcggccag gcctactgct acaacggcat gtgcctcacc    1560
   taccaggagc agtgccagca gctgtgggga cccggagccc gacctgcccc tgacctctgc    1620
   ttcgagaagg tgaatgtggc aggagacacc tttggaaact gtggaaagga catgaatggt    1680
   gaacacagga agtgcaacat gagagatgcg aagtgtggga agatccagtg tcagagctct    1740
   gaggcccggc ccctggagtc caacgcggtg cccattgaca ccactatcat catgaatggg    1800
   aggcagatcc agtgccgggg cacccacgtc taccgaggtc ctgaggagga gggtgacatg    1860
   ctggacccag ggctggtgat gactggaacc aagtgtggct acaaccatat ttgctttgag    1920
   gggcagtgca ggaacacctc cttctttgaa actgaaggct gtgggaagaa gtgcaatggc    1980
   catggggtct gtaacaacaa ccagaactgc cactgcctgc cgggctgggc cccgcccttc    2040
   tgcaacacac cgggccacgg gggcagtatc gacagtgggc ctatgccccc tgagagtgtg    2100
   ggtcctgtgg tagctggagt gttggtggcc atcttggtgc tggcggtcct catgctgatg    2160
   tactactgct gcagacagaa caacaaacta ggccaactca agccctcagc tctcccttcc    2220
   aagctgaggc aacagttcag ttgtcccttc agggtttctc agaacagcgg gactggtcat    2280
   gccaacccaa ctttcaagct gcagacgccc cagggcaagc gaaaggtgtt ccttgacttg    2340
   tgcgtacagg tgatcaacac tccggaaatc ctgcggaagc cctcccagcc tcctccccgg    2400
   ccccctccag attatctgcg tggtgggtcc ccacctgcac cactgccagc tcacctgagc    2460
   agggctgcta ggaactcccc agggcccggg tctcaaatag agaggacgga gtcgtccagg    2520
   aggcctcctc caagccggcc aattcccccc gcaccaaatt gcatcgtttc ccaggacttc    2580
   tccaggcctc ggccgcccca gaaggcactc ccggcaaacc cagtgccagg ccgcaggagc    2640
   ctccccaggc caggaggtgc atccccactg cggccccctg gtgctggccc tcagcagtcc    2700
   cggcctctgg cagcacttgc ccaaaaggtg agtccacggg aagccctcaa ggtgaaagct    2760
   ggtaccagag ggctccaggg gggcaggtgt agagttgaga aaacaaagca attcatgctt     2820
   cttgtggtct ggactgaact tccagaacaa aagccaaggg caaaacattc atgtttcttg    2880
   gtgcccgctt ga                                                        2892
<210> 6
   <211> 963
   <212> PRT
   <213> homo sapiens
<400> 6
   Met Pro Gly Gly Ala Gly Ala Ala Arg Leu Cys Leu Leu Ala Phe Ala
   1               5                   10                  15
   Leu Gln Pro Leu Arg Pro Arg Ala Ala Arg Glu Pro Gly Trp Thr Arg
               20                  25                  30
   Gly Ser Glu Glu Gly Ser Pro Lys Leu Gln His Glu Leu Ile Ile Pro
           35                  40                  45
   Gln Trp Lys Thr Ser Glu Ser Pro Val Arg Glu Lys His Pro Leu Lys
       50                  55                  60
   Ala Glu Leu Arg Val Met Ala Glu Gly Arg Glu Leu Ile Leu Asp Leu
   65                  70                  75                  80
   Glu Lys Asn Glu Gln Leu Phe Ala Pro Ser Tyr Thr Glu Thr His Tyr
                   85                  90                  95
   Thr Ser Ser Gly Asn Pro Gln Thr Thr Thr Arg Lys Leu Glu Asp His
               100                 105                 110
   Cys Phe Tyr His Gly Thr Val Arg Glu Thr Glu Leu Ser Ser Val Thr
           115                 120                 125
   Leu Ser Thr Cys Arg Gly Ile Arg Gly Leu Ile Thr Val Ser Ser Asn
   130 135 140
   Leu Ser Tyr Val Ile Glu Pro Leu Pro Asp Ser Lys Gly Gln His Leu
   145 150 155 160
   Ile Tyr Arg Ser Glu His Leu Lys Pro Pro Pro Gly Asn Cys Gly Phe
   165 170 175
   Glu His Ser Lys Pro Thr Thr Arg Asp Trp Ala Leu Gln Phe Thr Gln
   180 185 190
   Gln Thr Lys Lys Arg Pro Arg Arg Met Lys Arg Glu Asp Leu Asn Ser
   195 200 205
   Met Lys Tyr Val Glu Leu Tyr Leu Val Ala Asp Tyr Leu Glu Phe Gln
   210 215 220
   Lys Asn Arg Arg Asp Gln Asp Ala Thr Lys His Lys Leu Ile Glu Ile
   225 230 235 240
   Ala Asn Tyr Val Asp Lys Phe Tyr Arg Ser Leu Asn Ile Arg Ile Ala
   245 250 255
   Leu Val Gly Leu Glu Val Trp Thr His Gly Asn Met Cys Glu Val Ser
   260 265 270
   Glu Asn Pro Tyr Ser Thr Leu Trp Ser Phe Leu Ser Trp Arg Arg Lys
   275 280 285
   Leu Leu Ala Gln Lys Tyr His Asp Asn Ala Gln Leu Ile Thr Gly Met
   290 295 300
   Ser Phe His Gly Thr Thr Ile Gly Leu Ala Pro Leu Met Ala Met Cys
   305 310 315 320
   Ser Val Tyr Gln Ser Gly Gly Val Asn Met Asp His Ser Glu Asn Ala
   325 330 335
   Ile Gly Val Ala Ala Thr Met Ala His Glu Met Gly His Asn Phe Gly
   340 345 350
   Met Thr His Asp Ser Ala Asp Cys Cys Ser Ala Ser Ala Ala Asp Gly
   355 360 365
   Gly Cys Ile Met Ala Ala Ala Thr Gly His Pro Phe Pro Lys Val Phe
   370 375 380
   Asn Gly Cys Asn Arg Arg Glu Leu Asp Arg Tyr Leu Gln Ser Gly Gly
   385 390 395 400
   Gly Met Cys Leu Ser Asn Met Pro Asp Thr Arg Met Leu Tyr Gly Gly
   405 410 415
   Arg Arg Cys Gly Asn Gly Tyr Leu Glu Asp Gly Glu Glu Cys Asp Cys
   420 425 430
   Gly Glu Glu Glu Glu Cys Asn Asn Pro Cys Cys Asn Ala Ser Asn Cys
   435 440 445
   Thr Leu Arg Pro Gly Ala Glu Cys Ala His Gly Ser Cys Cys His Gln
   450 455 460
   Cys Lys Leu Leu Ala Pro Gly Thr Leu Cys Arg Glu Gln Ala Arg Gln
   465 470 475 480
   Cys Asp Leu Pro Glu Phe Cys Thr Gly Lys Ser Pro His Cys Pro Thr
   485 490 495
   Asn Phe Tyr Gln Met Asp Gly Thr Pro Cys Glu Gly Gly Gln Ala Tyr
   500 505 510
   Cys Tyr Asn Gly Met Cys Leu Thr Tyr Gln Glu Gln Cys Gln Gln Leu
   515 520 525
   Trp Gly Pro Gly Ala Arg Pro Ala Pro Asp Leu Cys Phe Glu Lys Val
   530 535 540
   Asn Val Ala Gly Asp Thr Phe Gly Asn Cys Gly Lys Asp Met Asn Gly
   545 550 555 560
   Glu His Arg Lys Cys Asn Met Arg Asp Ala Lys Cys Gly Lys Ile Gln
   565 570 575
   Cys Gln Ser Ser Glu Ala Arg Pro Leu Glu Ser Asn Ala Val Pro Ile
   580 585 590
   Asp Thr Thr Ile Ile Met Asn Gly Arg Gln Ile Gln Cys Arg Gly Thr
   595 600 605
   His Val Tyr Arg Gly Pro Glu Glu Glu Gly Asp Met Leu Asp Pro Gly
   610 615 620
   Leu Val Met Thr Gly Thr Lys Cys Gly Tyr Asn His Ile Cys Phe Glu
   625 630 635 640
   Gly Gln Cys Arg Asn Thr Ser Phe Phe Glu Thr Glu Gly Cys Gly Lys
   645 650 655
   Lys Cys Asn Gly His Gly Val Cys Asn Asn Asn Gln Asn Cys His Cys
   660 665 670
   Leu Pro Gly Trp Ala Pro Pro Phe Cys Asn Thr Pro Gly His Gly Gly
   675 680 685
   Ser Ile Asp Ser Gly Pro Met Pro Pro Glu Ser Val Gly Pro Val Val
   690 695 700
   Ala Gly Val Leu Val Ala Ile Leu Val Leu Ala Val Leu Met Leu Met
   705 710 715 720
   Tyr Tyr Cys Cys Arg Gln Asn Asn Lys Leu Gly Gln Leu Lys Pro Ser
   725 730 735
   Ala Leu Pro Ser Lys Leu Arg Gln Gln Phe Ser Cys Pro Phe Arg Val
   740 745 750
   Ser Gln Asn Ser Gly Thr Gly His Ala Asn Pro Thr Phe Lys Leu Gln
   755 760 765
   Thr Pro Gln Gly Lys Arg Lys Val Phe Leu Asp Leu Cys Val Gln Val
   770 775 780
   Ile Asn Thr Pro Glu Ile Leu Arg Lys Pro Ser Gln Pro Pro Pro Arg
   785 790 795 800
   Pro Pro Pro Asp Tyr Leu Arg Gly Gly Ser Pro Pro Ala Pro Leu Pro
   805 810 815
   Ala His Leu Ser Arg Ala Ala Arg Asn Ser Pro Gly Pro Gly Ser Gln
   820 825 830
   Ile Glu Arg Thr Glu Ser Ser Arg Arg Pro Pro Pro Ser Arg Pro Ile
   835 840 845
   Pro Pro Ala Pro Asn Cys Ile Val Ser Gln Asp Phe Ser Arg Pro Arg
   850 855 860
   Pro Pro Gln Lys Ala Leu Pro Ala Asn Pro Val Pro Gly Arg Arg Ser
   865 870 875 880
   Leu Pro Arg Pro Gly Gly Ala Ser Pro Leu Arg Pro Pro Gly Ala Gly
   885 890 895
   Pro Gln Gln Ser Arg Pro Leu Ala Ala Leu Ala Pro Lys Val Ser Pro
   900 905 910
   Arg Glu Ala Leu Lys Val Lys Ala Gly Thr Arg Gly Leu Gln Gly Gly
   915 920 925
   Arg Cys Arg Val Glu Lys Thr Lys Gln Phe Met Leu Leu Val Val Trp
   930 935 940
   Thr Glu Leu Pro Glu Gln Lys Pro Arg Ala Lys His Ser Cys Phe Leu
   945 950 955 960
   Val Pro Ala
<210> 7
   <211> 2868
   <212> DNA
   <213> homo sapiens
<400> 7
   atgccagggg gcgcaggcgc cgcccggctc tgcttgctgg cgtttgccct gcagcccctc   60
   cggccgcggg cggcgcggga gcctggatgg acaagaggaa gtgaggaagg cagccccaag 120
   ctgcagcatg aacttatcat acctcagtgg aagacttcag aaagccccgt gagagaaaag  180
   catccactca aagctgagct cagggtaatg gctgaggggc gagaactgat cctggacctg  240
   gagaagaatg agcaactttt tgctccttcc tacacagaaa cccattatac ttcaagtggt  300
   aaccctcaaa ccaccacacg gaaattggag gatcactgct tttaccacgg cacggtgagg  360
   gagacagaac tgtccagcgt cacgctcagc acttgccgag gaattagagg actgattacg  420
   gtgagcagca acctcagcta cgtcatcgag cccctccctg acagcaaggg ccaacacctt  480
   atttacagat ctgaacatct caagccgccc ccgggaaact gtgggttcga gcactccaag  540
   cccaccacca gggactgggc tcttcagttt acacaacaga ccaagaagcg acctcgcagg  600
   atgaaaaggg aagatttaaa ctccatgaag tatgtggagc tttacctcgt ggctgattat  660
   ttagagtttc agaagaatcg acgagaccag gacgccacca aacacaagct catagagatc  720
   gccaactatg ttgataagtt ttaccgatcc ttgaacatcc ggattgctct cgtgggcttg  780
   gaagtgtgga cccacgggaa catgtgtgaa gtttcagaga atccatattc taccctctgg  840
   tcctttctca gttggaggcg caagctgctt gcccagaagt accatgacaa cgcccaatta  900
   atcacgggca tgtccttcca cggcaccacc atcggcctgg cccccctcat ggccatgtgc  960
   tctgtgtacc agtctggagg agtcaacatg gaccactccg agaatgccat tggcgtggct 1020
   gccaccatgg cccacgagat gggccacaac tttggcatga cccatgattc tgcagattgc 1080
   tgctcggcca gtgcggctga tggtgggtgc atcatggcag ctgccactgg gcaccccttt 1140
   cccaaagtgt tcaatggatg caacaggagg gagctggaca ggtatctgca gtcaggtggt 1200
   ggaatgtgtc tctccaacat gccagacacc aggatgttgt atggaggccg gaggtgtggg 1260
   aacgggtatc tggaagatgg ggaagagtgt gactgtggag aagaagagga atgtaacaac 1320
   ccctgctgca atgcctctaa ttgtaccctg aggccggggg cggagtgtgc tcacggctcc 1380
   tgctgccacc agtgtaagct gttggctcct gggaccctgt gccgcgagca ggccaggcag 1440
   tgtgacctcc cggagttctg tacgggcaag tctccccact gccctaccaa cttctaccag 1500
   atggatggta ccccctgtga gggcggccag gcctactgct acaacggcat gtgcctcacc 1560
   taccaggagc agtgccagca gctgtgggga cccggagccc gacctgcccc tgacctctgc 1620
   ttcgagaagg tgaatgtggc aggagacacc tttggaaact gtggaaagga catgaatggt 1680
   gaacacagga agtgcaacat gagagatgcg aagtgtggga agatccagtg tcagagctct 1740
   gaggcccggc ccctggagtc caacgcggtg cccattgaca ccactatcat catgaatggg 1800
   aggcagatcc agtgccgggg cacccacgtc taccgaggtc ctgaggagga gggtgacatg 1860
   ctggacccag ggctggtgat gactggaacc aagtgtggct acaaccatat ttgctttgag 1920
   gggcagtgca ggaacacctc cttctttgaa actgaaggct gtgggaagaa gtgcaatggc 1980
   catggggtct gtaacaacaa ccagaactgc cactgcctgc cgggctgggc cccgcccttc 2040
   tgcaacacac cgggccacgg gggcagtatc gacagtgggc ctatgccccc tgagagtgtg 2100
   ggtcctgtgg tagctggagt gttggtggcc atcttggtgc tggcggtcct catgctgatg 2160
   tactactgct gcagacagaa caacaaacta ggccaactca agccctcagc tctcccttcc 2220
   aagctgaggc aacagttcag ttgtcccttc agggtttctc agaacagcgg gactggtcat 2280
   gccaacccaa ctttcaagct gcagacgccc cagggcaagc gaaaggtgat caacactccg 2340
   gaaatcctgc ggaagccctc ccagcctcct ccccggcccc ctccagatta tctgcgtggt 2400
   gggtccccac ctgcaccact gccagctcac ctgagcaggg ctgctaggaa ctccccaggg 2460
   cccgggtctc aaatagagag gacggagtcg tccaggaggc ctcctccaag ccggccaatt 2520
   ccccccgcac caaattgcat cgtttcccag gacttctcca ggcctcggcc gccccagaag 2580
   gcactcccgg caaacccagt gccaggccgc aggagcctcc ccaggccagg aggtgcatcc 2640
   ccactgcggc cccctggtgc tggccctcag cagtcccggc ctctggcagc acttgcccca 2700
   aaggtgagtc cacgggaagc cctcaaggtg aaagctggta ccagagggct ccaggggggc 2760
   aggtgtagag ttgagaaaac aaagcaattc atgcttcttg tggtctggac tgaacttcca 2820
   gaacaaaagc caagggcaaa acattcatgt ttcttggtgc ccgcttga               2868
<210> 8
   <211> 955
   <212> PRT
   <213> homo sapiens
<400> 8
   Met Pro Gly Gly Ala Gly Ala Ala Arg Leu Cys Leu Leu Ala Phe Ala
   1 5 10 15
   Leu Gln Pro Leu Arg Pro Arg Ala Ala Arg Glu Pro Gly Trp Thr Arg
   20 25 30
   Gly Ser Glu Glu Gly Ser Pro Lys Leu Gln His Glu Leu Ile Ile Pro
   35 40 45
   Gln Trp Lys Thr Ser Glu Ser Pro Val Arg Glu Lys His Pro Leu Lys
   50 55 60
   Ala Glu Leu Arg Val Met Ala Glu Gly Arg Glu Leu Ile Leu Asp Leu
   65 70 75 80
   Glu Lys Asn Glu Gln Leu Phe Ala Pro Ser Tyr Thr Glu Thr His Tyr
   85 90 95
   Thr Ser Ser Gly Asn Pro Gln Thr Thr Thr Arg Lys Leu Glu Asp His
   100 105 110
   Cys Phe Tyr His Gly Thr Val Arg Glu Thr Glu Leu Ser Ser Val Thr
   115 120 125
   Leu Ser Thr Cys Arg Gly Ile Arg Gly Leu Ile Thr Val Ser Ser Asn
   130 135 140
   Leu Ser Tyr Val Ile Glu Pro Leu Pro Asp Ser Lys Gly Gln His Leu
   145 150 155 160
   Ile Tyr Arg Ser Glu His Leu Lys Pro Pro Pro Gly Asn Cys Gly Phe
   165 170 175
   Glu His Ser Lys Pro Thr Thr Arg Asp Trp Ala Leu Gln Phe Thr Gln
   180 185 190
   Gln Thr Lys Lys Arg Pro Arg Arg Met Lys Arg Glu Asp Leu Asn Ser
   195 200 205
   Met Lys Tyr Val Glu Leu Tyr Leu Val Ala Asp Tyr Leu Glu Phe Gln
   210 215 220
   Lys Asn Arg Arg Asp Gln Asp Ala Thr Lys His Lys Leu Ile Glu Ile
   225 230 235 240
   Ala Asn Tyr Val Asp Lys Phe Tyr Arg Ser Leu Asn Ile Arg Ile Ala
   245 250 255
   Leu Val Gly Leu Glu Val Trp Thr His Gly Asn Met Cys Glu Val Ser
   260 265 270
   Glu Asn Pro Tyr Ser Thr Leu Trp Ser Phe Leu Ser Trp Arg Arg Lys
   275 280 285
   Leu Leu Ala Gln Lys Tyr His Asp Asn Ala Gln Leu Ile Thr Gly Met
   290 295 300
   Ser Phe His Gly Thr Thr Ile Gly Leu Ala Pro Leu Met Ala Met Cys
   305 310 315 320
   Ser Val Tyr Gln Ser Gly Gly Val Asn Met Asp His Ser Glu Asn Ala
   325 330 335
   Ile Gly Val Ala Ala Thr Met Ala His Glu Met Gly His Asn Phe Gly
   340 345 350
   Met Thr His Asp Ser Ala Asp Cys Cys Ser Ala Ser Ala Ala Asp Gly
   355 360 365
   Gly Cys Ile Met Ala Ala Ala Thr Gly His Pro Phe Pro Lys Val Phe
   370 375 380
   Asn Gly Cys Asn Arg Arg Glu Leu Asp Arg Tyr Leu Gln Ser Gly Gly
   385 390 395 400
   Gly Met Cys Leu Ser Asn Met Pro Asp Thr Arg Met Leu Tyr Gly Gly
   405 410 415
   Arg Arg Cys Gly Asn Gly Tyr Leu Glu Asp Gly Glu Glu Cys Asp Cys
   420 425 430
   Gly Glu Glu Glu Glu Cys Asn Asn Pro Cys Cys Asn Ala Ser Asn Cys
   435 440 445
   Thr Leu Arg Pro Gly Ala Glu Cys Ala His Gly Ser Cys Cys His Gln
       450                 455                 460
   Cys Lys Leu Leu Ala Pro Gly Thr Leu Cys Arg Glu Gln Ala Arg Gln
   465                 470                 475                 480
   Cys Asp Leu Pro Glu Phe Cys Thr Gly Lys Ser Pro His Cys Pro Thr
                   485                 490                 495
   Asn Phe Tyr Gln Met Asp Gly Thr Pro Cys Glu Gly Gly Gln Ala Tyr
               500                 505                 510
   Cys Tyr Asn Gly Met Cys Leu Thr Tyr Gln Glu Gln Cys Gln Gln Leu
           515                 520                 525
   Trp Gly Pro Gly Ala Arg Pro Ala Pro Asp Leu Cys Phe Glu Lys Val
       530                 535                 540
   Asn Val Ala Gly Asp Thr Phe Gly Asn Cys Gly Lys Asp Met Asn Gly
   545                 550                 555                 560
   Glu His Arg Lys Cys Asn Met Arg Asp Ala Lys Cys Gly Lys Ile Gln
                   565                 570                 575
   Cys Gln Ser Ser Glu Ala Arg Pro Leu Glu Ser Asn Ala Val Pro Ile
               580                 585                 590
   Asp Thr Thr Ile Ile Met Asn Gly Arg Gln Ile Gln Cys Arg Gly Thr
           595                 600                 605
   His Val Tyr Arg Gly Pro Glu Glu Glu Gly Asp Met Leu Asp Pro Gly
       610                 615                 620
   Leu Val Met Thr Gly Thr Lys Cys Gly Tyr Asn His Ile Cys Phe Glu
   625                 630                 635                 640
   Gly Gln Cys Arg Asn Thr Ser Phe Phe Glu Thr Glu Gly Cys Gly Lys
                   645                 650                 655
   Lys Cys Asn Gly His Gly Val Cys Asn Asn Asn Gln Asn Cys His Cys
               660                 665                 670
   Leu Pro Gly Trp Ala Pro Pro Phe Cys Asn Thr Pro Gly His Gly Gly
           675                 680                 685
   Ser Ile Asp Ser Gly Pro Met Pro Pro Glu Ser Val Gly Pro Val Val
       690                 695                 700
   Ala Gly Val Leu Val Ala Ile Leu Val Leu Ala Val Leu Met Leu Met
   705                 710                 715                 720
   Tyr Tyr Cys Cys Arg Gln Asn Asn Lys Leu Gly Gln Leu Lys Pro Ser
                   725                 730                 735
   Ala Leu Pro Ser Lys Leu Arg Gln Gln Phe Ser Cys Pro Phe Arg Val
               740                 745                 750
   Ser Gln Asn Ser Gly Thr Gly His Ala Asn Pro Thr Phe Lys Leu Gln
           755                 760                 765
   Thr Pro Gln Gly Lys Arg Lys Val Ile Asn Thr Pro Glu Ile Leu Arg
       770                 775                 780
   Lys Pro Ser Gln Pro Pro Pro Arg Pro Pro Pro Asp Tyr Leu Arg Gly
   785                 790                 795                 800
   Gly Ser Pro Pro Ala Pro Leu Pro Ala His Leu Ser Arg Ala Ala Arg
                   805                 810                 815
   Asn Ser Pro Gly Pro Gly Ser Gln Ile Glu Arg Thr Glu Ser Ser Arg
               820                 825                 830
   Arg Pro Pro Pro Ser Arg Pro Ile Pro Pro Ala Pro Asn Cys Ile Val
           835                 840                 845
   Ser Gln Asp Phe Ser Arg Pro Arg Pro Pro Gln Lys Ala Leu Pro Ala
       850                 855                 860
   Asn Pro Val Pro Gly Arg Arg Ser Leu Pro Arg Pro Gly Gly Ala Ser
   865                 870                 875                 880
   Pro Leu Arg Pro Pro Gly Ala Gly Pro Gln Gln Ser Arg Pro Leu Ala
                   885                 890                 895
   Ala Leu Ala Pro Lys Val Ser Pro Arg Glu Ala Leu Lys Val Lys Ala
                   900                     905                     910
   Gly Thr Arg Gly Leu Gln Gly Gly Arg Cys Arg Val Glu Lys Thr Lys
               915                     920                     925
   Gln Phe Met Leu Leu Val Val Trp Thr Glu Leu Pro Glu Gln Lys Pro
           930                     935                     940
   Arg Ala Lys His Ser Cys Phe Leu Val Pro Ala
   945                     950                     955
<210> 9
   <211> 3512
   <212> DNA
   <213> homo sapiens
<400> 9
   cacccgtccg ttctccatct ttcccgcctt cctcaaccct cgggtgacct tccttcttca      60
   tcctgcggag ccccggggct gagcggagcg tctcgacaga ggctggagcg ggtggggagg     120
   cgcggggcga gccggggggt tccagacgcg cctccaccgc cgggcagtgg gcaggtatgg      180
   ctgagggcgt gtgagcgccg agcgctaagg gccgccgcca ccatgccagg gggcgcaggc     240
   gccgcccggc tctgcttgct ggcgtttgcc ctgcagcccc tccggccgcg ggcggcgcgg     300
   gagcctggat ggacaagagg aagtgaggaa ggcagcccca agctgcagca tgaacttatc     360
   atacctcagt ggaagacttc agaaagcccc gtgagagaaa agcatccact caaagctgag     420
   ctcagggtaa tggctgaggg gcgagaactg atcctggacc tggagaagaa tgagcaactt     480
   tttgctcctt cctacacaga aacccattat acttcaagtg gtaaccctca aaccaccaca     540
   cggaaattgg aggatcactg cttttaccac ggcacggtga gggagacaga actgtccagc     600
   gtcacgctca gcacttgccg aggaattaga ggactgatta cggtgagcag caacctcagc     660
   tacgtcatcg agcccctccc tgacagcaag ggccaacacc ttatttacag atctgaacat     720
   ctcaagccgc ccccgggaaa ctgtgggttc gagcactcca agcccaccac cagggactgg     780
   gctcttcagt ttacacaaca gaccaagaag cgacctcgca ggatgaaaag ggaagattta     840
   aactccatga agtatgtgga gctttacctc gtggctgatt atttagagtt tcagaagaat     900
   cgacgagacc aggacgccac caaacacaag ctcatagaga tcgccaacta tgttgataag     960
   ttttaccgat ccttgaacat ccggattgct ctcgtgggct tggaagtgtg gacccacggg    1020
   aacatgtgtg aagtttcaga gaatccatat tctaccctct ggtcctttct cagttggagg    1080
   cgcaagctgc ttgcccagaa gtaccatgac aacgcccaat taatcacggg catgtccttc    1140
   cacggcacca ccatcggcct ggcccccctc atggccatgt gctctgtgta ccagtctgga    1200
   ggagtcaaca tggaccactc cgagaatgcc attggcgtgg ctgccaccat ggcccacgag    1260
   atgggccaca actttggcat gacccatgat tctgcagatt gctgctcggc cagtgcggct    1320
   gatggtgggt gcatcatggc agctgccact gggcacccct ttcccaaagt gttcaatgga    1380
   tgcaacagga gggagctgga caggtatctg cagtcaggtg gtggaatgtg tctctccaac    1440
   atgccagaca ccaggatgtt gtatggaggc cggaggtgtg ggaacgggta tctggaagat    1500
   ggggaagagt gtgactgtgg agaagaagag gaatgtaaca acccctgctg caatgcctct     1560
   aattgtaccc tgaggccggg ggcggagtgt gctcacggct cctgctgcca ccagtgtaag    1620
   ctgttggctc ctgggaccct gtgccgcgag caggccaggc agtgtgacct cccggagttc    1680
   tgtacgggca agtctcccca ctgccctacc aacttctacc agatggatgg taccccctgt    1740
   gagggcggcc aggcctactg ctacaacggc atgtgcctca cctaccagga gcagtgccag    1800
   cagctgtggg gacccggagc ccgacctgcc cctgacctct gcttcgagaa ggtgaatgtg    1860
   gcaggagaca cctttggaaa ctgtggaaag gacatgaatg gtgaacacag gaagtgcaac   1920
   atgagagatg cgaagtgtgg gaagatccag tgtcagagct ctgaggcccg gcccctggag    1980
   tccaacgcgg tgcccattga caccactatc atcatgaatg ggaggcagat ccagtgccgg    2040
   ggcacccacg tctaccgagg tcctgaggag gagggtgaca tgctggaccc agggctggtg    2100
   atgactggaa ccaagtgtgg ctacaaccat atttgctttg aggggcagtg caggaacacc    2160
   tccttctttg aaactgaagg ctgtgggaag aagtgcaatg gccatggggt ctgtaacaac    2220
   aaccagaact gccactgcct gccgggctgg gccccgccct tctgcaacac accgggccac    2280
   gggggcagta tcgacagtgg gcctatgccc cctgagagtg tgggtcctgt ggtagctgga    2340
   gtgttggtgg ccatcttggt gctggcggtc ctcatgctga tgtactactg ctgcagacag    2400
   aacaacaaac taggccaact caagccctca gctctccctt ccaagctgag gcaacagttc    2460
   agttgtccct tcagggtttc tcagaacagc gggactggtc atgccaaccc aactttcaag    2520
   ctgcagacgc cccagggcaa gcgaaaggtg ttccttgact tgtgcgtaca ggtgatcaac 2580
   actccggaaa tcctgcggaa gccctcccag cctcctcccc ggccccctcc agattatctg 2640
   cgtggtgggt ccccacctgc accactgcca gctcacctga gcagggctgc taggaactcc 2700
   ccagggcccg ggtctcaaat agagaggacg gagtcgtcca ggaggcctcc tccaagccgg 2760
   ccaattcccc ccgcaccaaa ttgcatcgtt tcccaggact tctccaggcc tcggccgccc 2820
   cagaaggcac tcccggcaaa cccagtgcca ggccgcagga gcctccccag gccaggaggt 2880
   gcatccccac tgcggccccc tggtgctggc cctcagcagt cccggcctct ggcagcactt 2940
   gccccaaagg tgagtccacg ggaagccctc aaggtgaaag ctggtaccag agggctccag 3000
   gggggcaggt gtagagttga gaaaacaaag caattcatgc ttcttgtggt ctggactgaa 3060
   cttccagaac aaaagccaag ggcaaaacat tcatgtttct tggtgcccgc ttgactgtgg 3120
   agttttggct tcatgtgaaa ggtgattctt agaatcctga gctgtggtgg cttcagtcct 3180
   gcccctgcac ctgacctggg gagggaccct gagcaagtcc ctcttgagtc tgtttcctca 3240
   tttgtacaga ggctatgatg agcagtagta ccagccccat caggttgttg tgaagagcaa 3300
   ggaagtgcat tagtagaacc acctggcctg cgggaggctg tgtacacttg gcccctyctc 3360
   gtacttcttc tgtgtggctg acagtctcag tttgccagtc ttggcartca ggtcagaaag 3420
   ggatgaggtg aagaagctgg ctkrmcatgc tgtattccca cgaggcattg agaaaactat 3480
   ggactgtgct gctgccttar cagggaaggg aa 3512